(19) Europäisches Patentamt
European Patent Office
Office européen des brevets

(11) Publication number: 0 073 026
B1

(12) **EUROPEAN PATENT SPECIFICATION**

(45) Date of publication of patent specification: 19.03.86

(51) Int. Cl.⁴: C 07 J 31/00, A 61 K 31/565

(21) Application number: 82107589.2

(22) Date of filing: 19.08.82

# (54) 17,17-Bis(substituted thio)-3-ketoandrostenes and pharmaceutical compositions.

(30) Priority: 20.08.81 US 294680

(43) Date of publication of application:
02.03.83 Bulletin 83/09

(45) Publication of the grant of the patent:
19.03.86 Bulletin 86/12

(84) Designated Contracting States:
AT BE CH DE FR GB IT LI LU NL SE

(56) References cited:
US-A-2 840 577
US-A-2 949 477
US-A-4 094 840

(73) Proprietor: E.R. Squibb & Sons, Inc.
Lawrenceville-Princeton Road
Princeton, N.J. 08540 (US)

(72) Inventor: Varma, Ravi K.
293 Deer Run Drive
Belle Mead New Jersey (US)

(74) Representative: Vossius Vossius Tauchner
Heunemann Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 073 026 B1

Courier Press, Leamington Spa, England.

**Description**

This invention relates to 17,17-bis(substituted thio)-3-ketoandrostenes having in the 17-position the substituents $R_1$—S— and $R_2$—S— wherein $R_1$ and $R_2$ are the same or different and each is alkyl, cycloalkyl or aryl.

Those 3-ketoandrostenes having in the 17-position the substituents $R_1$—S— and $R_2$—S— wherein $R_1$ and $R_2$ are different groups and each is alkyl, cycloalkyl or aryl are novel steroids, and as such, form an integral part of this invention.

A novel process for preparing 3-ketoandrostenes having in the 17-position the substituents $R_1$—S— and $R_2$—S— wherein $R_1$ and $R_2$ are the same is also disclosed herein.

Exemplary of the above-described antiinflammatory 3-ketoandrostenes are those steroids having the formula

I

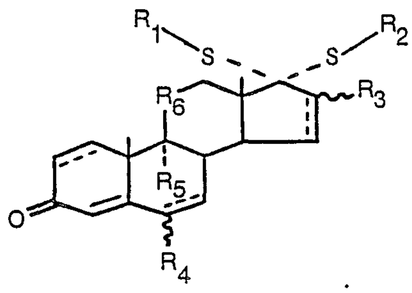

In formula I, and throughout the specification, the symbols are as defined below.

$R_1$ and $R_2$ are the same or different and each is alkyl, cycloalkyl or aryl;

$R_3$ is hydrogen, hydroxy, alkoxy, aryloxy, alkylthio, arylthio,

O
||
alkyl-C—O—

or halogen;

$R_4$ is hydrogen, methyl, hydroxy,

O
||
alkyl-C—O—,

or halogen;

$R_5$ is hydrogen or halogen; and

$R_6$ is carbonyl or β-hydroxymethylene.

A broken line in the 1,2-, 6,7- and 15,16-position of a structural formula in this specification indicates the optional presence of ethylenic unsaturation.

The term "aryl", as used throughout the specification either individually or as part of a larger group, refers to phenyl or phenyl substituted with one or two alkyl, alkoxy or halogen groups.

The term "halogen", as used throughout the specification either individually or as part of a larger group, refers to fluorine, chlorine, bromine and iodine.

The terms "alkyl" and "alkoxy", as used throughout the specification either individually or as part of a larger group, refer to groups having 1 to 12 carbon atoms.

The term "cycloalkyl", as used throughout the specification either individually or as part of a larger group, refers to groups having 3, 4, 5, 6 or 7 carbon atoms.

The term "androstene", as used throughout the specification, refers to androstanes having ethylenic unsaturation in one or more positions. Exemplary of androstenes specifically contemplated are $\Delta^4$-androstenes, $\Delta^{1,4}$-androstadienes, $\Delta^{4,6}$-androstadienes, $\Delta^{1,4,6}$-androstatrienes, $\Delta^{1,4,15}$-androstatrienes, $\Delta^{4,6,15}$-androstatrienes and $\Delta^{1,4,6,15}$-androstatetraenes.

As set forth above, the 3-ketoandrostenes having in the 17-position the substituents $R_1$—S— and $R_2$—S— wherein $R_1$ and $R_2$ are different groups are novel steroids that form an integral part of this invention.

The compounds of formula I wherein $R_1$ and $R_2$ are the same and $R_3$ is hydroxy, alkoxy, aryloxy, alkylthio, arylthio,

O
||
alkyl-C—O—,

or halogen are also novel compounds that form an integral part of this invention.

3-Ketoandrostenes having in the 17-position the substituents $R_1$—S— and $R_2$—S— are topical antiinflammatory agents that can be used to treat skin conditions such as dermatitis, psoriasis, sunbuurn, eczema, neurodermatitis, or anogenital pruritus, and in inhalation therapy for topical treatment of allergy and asthma.

For the treatment of skin conditions, the steroids useful in the method of this invention may be administered in a conventional pharmaceutical carrier in the form of a cream, ointment, lotion or the like. The steroids will preferably be used in the range of 0.01 to 5.0% by weight of the vehicle, preferably 0.05 to 2.0% by weight of the vehicle.

For the topical treatment of allergy and asthma the steroids useful in the method of this invention may be administered in the conventional manner, *e.g.,* as solid medicament which has been atomized. United States patents 3,948,264 and 4,147,166 are exemplary of the literature which describes devices that can be used to administer solid medicaments for inhalation therapy.

The preparation of the 3-keto-17,17-bis(substituted thio)androstenes of this invention is described below with specific reference to the steroid of formula I.

The steroids of formula I can be prepared utilizing androstenes having the formula

II

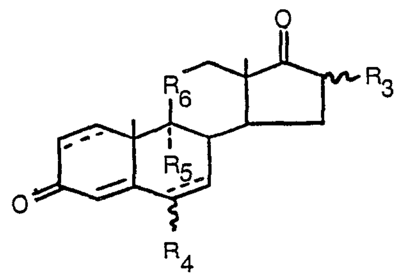

,

as starting materials.

Reaction of an androstene of formula II with a thiol having the formula

III $R_1$—SH

in the presence of a Lewis acid (*e.g.,* boron trifluoride etherate), yields a product having the formula

IV

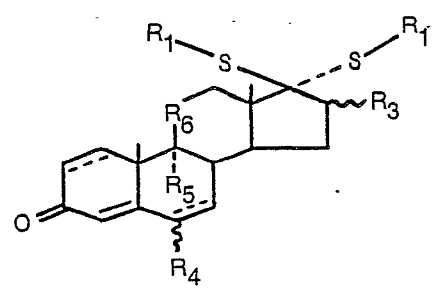

The reaction can be run in an organic solvent (*e.g.,* a halogenated hydrocarbon), or a mixture of organic solvents. The use of glacial acetic acid as the sole solvent, or in admixture with other solvents, improves yields. Reactions can be conveniently run at room temperature, preferably in an inert atmosphere (*e.g.,* argon or nitrogen). Better yields can be obtained with relatively short reaction times (less than 1 hour).

It has been found that the yields of a reaction of a steroid of formula II with a thiol of formula III can be improved by adding a small amount of a dimethylformamide dialkyl acetal (preferably dimethylformamide dimethyl acetal). The use of such an agent improves both the yield and rate of the reaction, and tends to suppress the reaction of the thiol with the A-ring double bond and ketone functions.

To prepare the steroids of formula I wherein $R_1$ and $R_2$ are different, an androstene of formula IV is first converted to the corresponding androstene having the formula

V

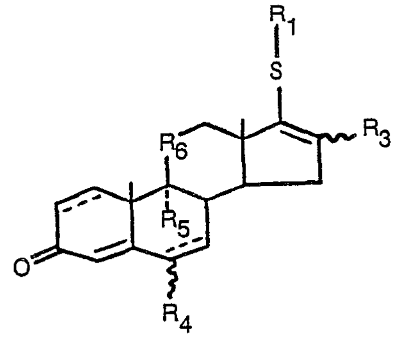

by simply heating the steroid, either neat or in an inert solvent (*e.g.,* diethylbenzene or dichlorobenzene).

Alternatively, compounds of formula V, wherein $R_3$ is chlorine, bromine, alkylthio, or arylthio can be prepared from the corresponding steroid of formula V wherein $R_3$ is hydrogen; *i.e.,* a steroid having the formula

VI

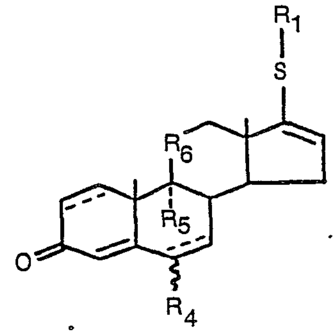

Utilizing the procedure described in United States patent 4,265,815 issued May 5, 1981, a steroid of formula V wherein $R_3$ is chlorine or bromine can be obtained by reacting a steroid of formula VI with the appropriate N-halosuccinimide, or with chlorine or bromine, preferably in a halogenated hydrocarbon solvent. Steroids of formula V wherein $R_3$ is alkylthio or arylthio can be obtained by reacting the corresponding steroid of formula VI with an alkyl or aryl sulfenyl halide, preferably in a halogenated hydrocarbon solvent.

Reaction of a steroid of formula V with a thiol having the formula

VII $R_2$—SH

yields the corresponding steroid having the formula

VIII

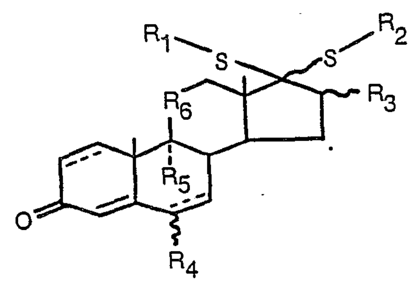

as a mixture of isomers. The reaction is run in the presence of a Lewis acid (*e.g.,* boron trifluoride etherate) and is preferably run at a reduced temperature (*i.e.,* about −20°C to −100°C). When the reaction is run at a reduced temperature (*i.e.,* about −20°C to −100°C), it is stereospecific, and yields a steroid having the formula

IX

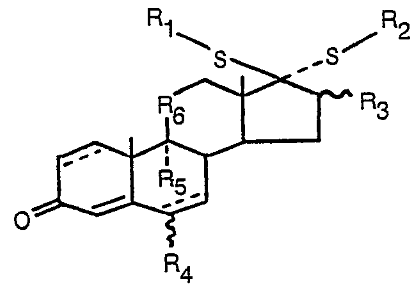

The 11-hydroxyl group of a steroid of formula V may be protected before its reaction with a thiol of formula VII. An exemplary family of protecting groups is the acyl family, *e.g.,* alkanoyl groups such as acetyl. Means for protection and deprotection of the 11-hydroxyl group are well-known in the art. When preparing a compound of formula VIII or IX from an androstene-3,17-dione of formula II, it may be desirable to protect the 11-hydroxyl group as the first step of the synthesis.

The steroids of formula I having ethylenic unsaturation in the 15,16-position can be prepared from the corresponding 16-haloandrostene. Refluxing the 16-haloandrostene in an organic solvent in the presence of 1,5-diazabicyclo (5.4.0) undec-5-ene yields the desired 15,16-unsaturation. Alternatively, the steroids of formula I having ethylenic unsaturation in the 15,16-position can be prepared from the corresponding 16-hydroxyandrostene. Dehydrating the 16-hydroxyandrostene, using a dehydrating agent such as thionyl chloride, yields the desired 15,16-unsaturation.

The starting androstenes of formula II can be prepared by treating the corresponding pregnene having the formula

X

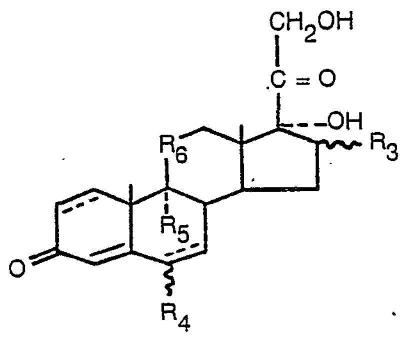

with sodium bismuthate in the presence of an acid, such as acetic acid.

Alternatively, the starting androstenes of formula II wherein $R_3$ is hydroxy or

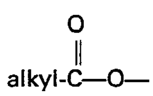

can be prepared by oxidation of the corresponding androstene having the formula

XI

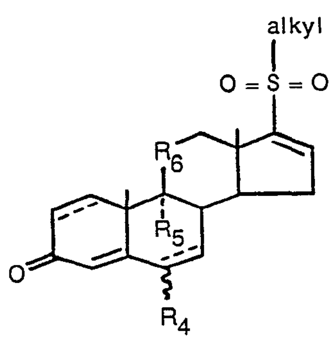

with potassium permanganate in the presence of formic acid. The oxidation reaction yields the corresponding 16α-hydroxyandrostene-3,17-dione. This can be acylated using art-recognized procedures to yield the corresponding 17-alkanoyloxy derivative.

The following examples are specific embodiments of this invention.

Example 1

(11β,16α)-9-Fluoro-11,16-dihydroxy-17,17-bis(methylthio)androsta-1,4-dien-3-one

A) 9-Fluoro-11β,16α-dihydroxyandrosta-1,4-diene-3,17-dione

A solution of 9-fluoro-11β-hydroxy-17-(methylsulfonyl)androsta-1,4,16-triene-3-one (760 mg) in purified acetone (250 ml) is stirred in a bath at −3° to 0°C and 3.0 ml of 10% (w/v) formic acid is added followed dropwise by a solution of potassium permanganate (540 mg) in purified acetone (250 ml). After 2.0 hours a few drops of 30% hydrogen peroxide are added to decompose any excess permanganate. The mixture is then filtered through a bed of anhydrous magnesium sulfate which is subsequently washed with small amounts of acetone. The filtrate and the washings are combined and concentrated *in vacuo*. The concentrate is diluted with water (500 ml) and extracted with chloroform. The chloroform extracts are combined, washed with water, dried ($MgSO_4$ anhydrous) and evaporated to afford the title compound (550 mg) as a crystalline solid. Crystallization of this from acetone-hexane gives the analytical specimen, melting point 227—228°C, with consistent spectral data.

B) (11β,16α)-9-Fluoro-11,16-dihydroxy-17,17-bis(methylthio)androsta-1,4-dien-3-one

To an ice-cold solution of 9-fluoro-11β,16α-dihydroxyandrosta-1,4-diene-3,17-dione (300 mg) in a mixture of dichloromethane (6.0 ml) and acetic acid (6.0 ml) containing methanethiol (0.3 ml) is added boron trifluoride etherate (0.3 ml). The solution is then stirred at room temperature for 35 minutes. It is then poured into water and extracted with chloroform. The chloroform solution is washed with a dilute sodium bicarbonate solution and water, dried ($MgSO_4$ anhydrous) and is evaporated to afford a glassy solid. The solid is chromatographed on a column of silica gel (10 g) eluting the column with chloroform and chloroform-ethyl acetate mixtures to afford the homogeneous title compound as a solid (170 mg). One recrystallization of this from acetone-hexane and drying (100°C, 0.4mbar (0.3 mm of Hg), 10 hours) gives the analytical specimen (143 mg) melting point 261—262°C, *dec.* with consistent spectral data.

| | | | | |
|---|---|---|---|---|
| Anal. Calcd. for $C_{21}H_{29}FO_3S_2$: | C, 61.13; | H, 7.08; | F, 4.61; | S, 15.54 |
| Found: | C, 61.29; | H, 7.14; | F, 4.60; | S, 15.39 |

Example 2

17, 17-Bis(ethylthio)-11β-hydroxyandrosta-1,4-dien-3-one

A solution of 11β-hydroxyandrosta-1,4-diene-3,17-dione (1.0 g) in acetic acid (25 ml) containing ethanethiol (1.5 ml) and boron trifluoride etherate (2.0 ml) is stirred at room temperature for 45 minutes. The mixture is then added to water (200 ml) and is extracted with chloroform. The chloroform extracts are combined, washed with a saturated sodium bicarbonate solution and water dried ($MgSO_4$ anhydrous) and evaporated *in vacuo* to a gummy residue. The residue is chromatographed on a column of silica gel (30 g) eluting successively with chloroform-hexane (4:1), chloroform and chloroform-ethyl acetate (9:1 and 4:1) to isolate respectively the overreacted steroid (about 100 mg), the homogeneous (tlc) title compound (1.2 g) and unreacted starting steroid (about 75 mg). One crystallization of the 1.2 g of solid from ethyl acetate-hexane affords flakes of the analytical specimen of the title compound (800 mg) melting point 163—165°C with consistent spectral data, after drying at 100°C at 0.4 mbar (0.3 mm of Hg) for 7 hours.

| | | | |
|---|---|---|---|
| Anal. Calcd. for $C_{23}H_{34}O_2S_2$: | C, 67.93; | H, 8.43; | S, 15.77 |
| Found: | C, 67.84; | H, 8.45; | S, 15.69 |

Example 3

(11β)-9-Fluoro-11-hydroxy-17,17-bis(propylthio)-androsta-1,4-dien-3-one

Boron trifluoride etherate (3.58 g) is added to a solution of 9-fluoro-11β-hydroxyandrosta-1,4-diene-3,17-dione (1.5 g), n-propanethiol (1.77 g) and dimethylformamide-dimethylacetal (1.52 g) in glacial acetic acid (35 ml). After 2 hours, the reaction mixture is poured into water (300 ml) and the products are extracted into chloroform. The chloroform extracts are combined, washed with water, a dilute $NaHCO_3$ solution and water, dried ($MgSO_4$ anhydrous) and evaporated to afford the product as a solid (1.8 g). This is chromatographed on a column of silica gel (25 g) eluting with chloroform-hexane (4:1), chloroform and chloroform-ethyl acetate (95:5 and 9:1) to isolate the title compound (1.20 g) and unreacted starting material (400 mg). Two recrystallizations of the 1.2 g material from ethyl acetate-hexane and drying (105°C, 0.4 mbar (0.3 mm of Hg), 6.0 hours) gives the analytical specimen (900 mg) melting point 235—237°C, with constant spectral data.

| | | | | | |
|---|---|---|---|---|---|
| Anal. for $C_{25}H_{37}FO_2S_2$: | Calc'd: | C, 66.33; | H, 8.22; | S, 14.16; | F, 4.20 |
| Found: | | C, 66.34; | H, 8.29; | S, 14.06; | F, 4.29 |

Example 4

(11β)-17,17-Bis(butylthio)-9-fluoro-11-hydroxyandrosta-1,4-dien-3-one

To a solution of 9-fluoro-11β-hydroxyandrosta-1,4-diene-3,17-dioine (4.2 g) in glacial acetic acid (120 ml) containing n-butanethiol (5.0 ml) is added boron trifluoride etherate (2.5 ml). A blue color develops after about 1.0 hour. The blue reaction mixture is poured into water (700 ml) and extracted with chloroform. The chloroform extracts are combined, washed with a saturated sodium bicarbonate solution and water, dried

($MgSO_4$, anhydrous) and evaporated to a gummy residue. This is chromatographed over silica gel (70 g), eluting the column with chloroform-hexane (8:2), chloroform and chloroform-ethyl acetate (9:1) and 8:2) to isolate successively the over-reacted steroidal material contaminated with other thiol-derived products, the title compound (400 mg) and starting steroid (2.5 g). Two recrystallizations of the 400 mg from ethyl acetate-hexane gives the analytical specimen of the title compound (125 mg), melting point 160—162°C, with consistent spectral data.

| | | | | |
|---|---|---|---|---|
| Anal. for $C_{27}H_{41}FO_2S_1$: | C, 67.45; | H, 8.59; | F, 3.95; | S, 13.34 |
| Found: | C, 67.37; | H, 8.61; | F, 3.86; | S, 13.29 |

## Example 5

### (11β,16α)-17,17-Bis(ethylthio)-9-fluoro-11-hydroxy-16-methoxyandrosta-1,4-dien-3-one

A) 9-Fluoro-11β-hydroxy-16α-methoxyandrosta-1,4-dien-3,17-one

9-Fluoro-11β,17-21-trihydroxy-16α-methoxypregna-1,4-diene-3,20-dione (4.0 g) is dissolved in 50% acetic acid (300 ml) by warming. The solution is cooled to room temperature, sodium bismuthate (25 g) is added and the mixture is stirred at 55°C (oil bath temperature) for 24 hours. The resulting mixture is filtered through a bed of Hyflo and washed with a small amount of warm 50% acetic acid. The filtrate is concentrated to 50 ml *in vacuo*, diluted with 200 ml of 20% hydrochloric acid and extracted with chloroform. The chloroform solution is washed with water, dried over anhydrous $Na_2SO_4$ and evaporated *in vacuo* to give a foam (3.0 g). This is dissolved in chloroform and chromatographed on a 30 g-silica gel column, eluting successively with chloroform and chloroform-ethyl acetate (95:5, 9:1 and 8:2) to give 1.4 g of a slightly impure title compound. This is rinsed with chloroform-hexane (1:1) to give 1.0 g of thin-layer chromatography (tlc)-homogeneous solid, melting point 204—210°C with consistent spectral data.

B) (11β,16α)-17,17-Bis(ethylthio)-9-fluoro-11-hydroxy-16-methoxyandrosta-1,4-dien-3-one

A solution of 1.0 g of 9-fluoro-11β-hydroxy-16α-methoxyandrosta-1,4-dien-3,17-dione, 1.06 ml of ethanethiol, 1.78 ml of boron trifluoride etherate and 853 mg of N,N-dimethylformamide dimethyl acetal in 28 ml of glacial acetic acid is stirred at room temperature under nitrogen for 1.5 hour. The resulting solution is diluted with chloroform, washed with water, saturated $NaHCO_3$ solution and water, dried over anhydrous $Na_2SO_4$ and evaporated *in vacuo* to give a foam. This is dissolved in chloroform-hexane (9:1) and chromatographed on a 35 g-silica gel column, eluting successively with chloroform-hexane (9:1), chloroform, chloroform-ethyl acetate (95:5) and chloroform-methanol (9:1) to give an over-reacted steroidal product (130 mg), 9-fluoro-11β-hydroxy-16α-methoxyandrosta-1,4-diene-3,20-dione (230 mg) and title compound (510 mg). The title compound is recrystallized from acetone-hexane to give 385 mg of an analytical specimen, melting point 234—239°C, with consistent spectral data.

| | | | | |
|---|---|---|---|---|
| Anal. Calcd. for $C_{24}H_{35}FO_3S_2$: | C, 63.40; | H, 7.76; | F, 4.18; | S, 14.11 |
| Found: | C, 63.26; | H, 7.78; | F, 4.21; | S, 14.00 |

## Example 6

### 9-Fluoro-11β-hydroxy-17,17-bis(phenylthio)androsta-1,4-dien-3-one

A solution of 9.0 g of 9-fluoro-11β-hydroxyandrosta-1,4-diene-3,17-dione in 50 ml of dichloromethane and 50 ml of glacial acetic acid is stirred with 18.68 g of thiophenol and 7.5 ml of boron trifluoride etherate at room temperature under nitrogen. After 50 minutes the solution is diluted with 350 ml of chloroform. The chloroform solution is washed with water, saturated $NaHCO_3$ solution and water, dried over anhydrous $Na_2SO_4$ and evaporated *in vacuo* to give 11.6 g of an oil. This is dissolved in 1:3 hexane-chloroform and chromatographed on a 200 g-silica gel column. Elution with 1:3 hexane-chloroform and chloroform gives 3.5 g of a homogeneous material. Crystallization from chloroform-methanol gives 2.0 g of the title compound, melting point 249—250°C, *dec.* with consistent spectral data.

| | | | | |
|---|---|---|---|---|
| Anal. Calcd. for $C_{31}H_{33}FO_2S_2$: | C, 71.50; | H, 6.39; | F, 3.65; | S, 12.32 |
| Found: | C, 71.66; | H, 6.49; | F, 3.92; | S, 12.41 |

## Example 7

### 9-Fluoro-11β-hydroxy-17,17-bis(methylthio)androsta-1,4-dien-3-one

A solution of 9-fluoro-11β-hydroxyandrosta-1,4-diene-3,20-dione (2.0 g) in glacial acetic acid (25 ml) is mixed at room temperature with a solution of methanethiol (2.4 g) in dichloromethane (16 ml) and boron trifluoride etherate (0.5 ml). After 1.5 hours, the mixture is poured into water and diluted with chloroform. The organic layer is then separated, washed with a dilute sodium bicarbonate solution and water, dried ($MgSO_4$ anhydrous) and evaporated *in vacuo*. The residue is absorbed on a column of silica gel (50 g). Elution of the column with chloroform removes the non-steroidal impurities and a product resulting from thiol addition to the A-ring. Subsequent elution with chloroform affords the homogeneous product as a solid (957 mg). Elution with chloroform-ethyl acetate (95:5) affords the unreacted steroid (345 mg). A specimen of the 957 mg of solid is crystallized from chloroform-methanol to afford the analytical specimen of product, melting point 305°C, *dec.* with consistent spectral data.

| | | | | |
|---|---|---|---|---|
| Anal. Calcd. for $C_{21}H_{29}FO_2S_2$: | C, 63.60; | H, 7.37; | F, 4.79; | S, 16.17 |
| Found: | C, 63.48; | H, 7.21; | F, 4.95; | S, 16.21 |

Example 8

17,17-Bis(ethylthio)-9-fluoro-11β-hydroxyandrosta-1,4-dien-3-one

A solution of 9.5 g of 9-fluoro-11β-hydroxyandrosta-1,4-diene-3,17-dione in 50 ml of dichloromethane and 50 ml of glacial acetic acid is stirred with 11.2 g of ethanethiol and 7.5 ml of boron trifluoride etherate at room temperature under nitrogen. After 1.5 hours the solution is diluted with 350 ml fo chloroform. The chloroform solution is washed with water, saturated $NaHCO_3$ solution and water, dried over anhydrous $Na_2SO_4$ and evaporated *in vacuo* to give 11 g of a foamy solid. This is dissolved in hexane-chloroform (2:1) and chromatographed on a 200 g-silica gel column. Elution with hexane-chloroform (2.1 and 1:1) gives 2.1 g of a homogeneous material. Crystallization from acetone-hexane gives 1.05 g of the title compound, melting point 276—277°C, *dec.*, with consistent spectral data.

Anal. Calcd. for $C_{23}H_{33}FO_2S_2$: C, 65.05; H, 7.83; F, 4.47; S, 15.10
Found: C, 65.31; H, 7.80; F, 4.71; S, 15.01

Example 9

9-Fluoro-11β-hydroxy-17,17-bis[(4-methoxyphenyl)thio]androsta-1,4-dien-3-one

To a solution of 9-fluoro-11β-hydroxyandrosta-1,4-diene-3,17-dione (3.18 g) in a mixture of dry dichloromethane (40 ml) and glacial acetic acid (40 ml) containing p-methoxybenzenethiol (5.6 g) is added boron trifluoride etherate (3.0 ml) and the resulting solution is stirred for 1.5 hours. It is then poured into water (500 ml) and extracted with chloroform. The chloroform extracts are combined, washed with saturated sodium bicarbonate solution and water, dried ($MgSO_4$) and concentrated *in vacuo* to a syrupy residue. This is absorbed on a column of silica gel (50 g) made up in chloroform-hexane (1:1) and the column is eluted successively with chloroform-hexane (1:1), chloroform and chloroform-ethyl acetate mixtures (95:5 and 90:10) to elute successively p-methoxybenzenethiol contaminated with some steroidal impurities, the title compound (3.0 g), a small amount of an unidentified compound and unreacted starting material (1.0 g). The 3.0 g of material is refluxed with ethyl acetate (30 ml), cooled and filtered to leave the analytical specimen (dried at 0.4 mbar (0.3 mm of Hg), 100°C', 18 hours) of the title compound (2.8 g), melting point 209—211°C, with consistent spectral data.

Anal. for $C_{33}H_{37}FO_4S_2$: Calcd: C, 68.25; H, 6.42; F, 3.27; S, 11.04
Found: C, 68.46; H, 6.63; F, 3.25; S, 11.20

Example 10

17-(Ethylthio)-9-fluoro-11β-hydroxy-17-(methylthio)-androsta-1,4-dien-3-one

A) 11β-Acetyloxy-9-fluoroandrosta-1,4-diene-3,17-dione

A solution of 9-fluoro-11β-hydroxyandrosta-1,4-diene-3,17-dione (5.0 g) in a mixture of acetic acid (60 ml) and acetic anhydride (60 ml) containing *p*-toluenesulfonic acid (2.5 g) is maintained at room temperature for eighteen hours. Sodium acetate (2.5 g) is added and the mixture is concentrated *in vacuo* at 35—40°C. The residue is diluted with water (150 ml) and the solid that separates is isolated by filtration, washed with water and dried *in vacuo* to afford the title compound as a solid (5.0 g) with consistent spectral data. An examination (silica gel, chloroform: ethyl acetate, 95:5) reveals the presence of a small amount of starting steroid as the only significant impurity. This material is used without purification in the next step. A specimen crystallized from acetone-hexane melts at 173—174°C.

B) 11β-Acetyloxy-17,17-bis(methylthio)-9-fluoroandrosta-1,4-diene-3-one

To a solution of 11β-acetyloxy-9-fluoroandrosta-1,4-diene-3-one (5.0 g) in a mixture of acetic acid (25 ml) and dichloromethane (25 ml) containing methanethiol (2.5 ml) is added distilled boron trifluoride etherate (0.5 ml) and the mixture is stirred for one hour. It is then added to water (150 ml) and is extracted with chloroform. The chloroform solution is washed with water, saturated $NaHCO_3$ solution and water, dried ($MgSO_4$ anhydrous) and evaporated. The residue is absorbed on a column of silica gel (30 g). Successive elutions of the column with chloroform-hexane (4:1), chloroform and chloroform-ethyl acetate (95:5 and 9:1) afford overreacted steroid containing thiol-derived impurities (3.0 g), the title compound (1.7 g) and unreacted starting material. Crystallization of the 1.7 g material from acetone-hexane gives 1.2 g of material, melting point 220—222°C, with consistent spectral data.

C) 11β-Acetyloxy-9-fluoro-17-(methylthio)-androsta-1,4,16-triene-3-one

11β-Acetyloxy-17,17-bis(methylthio)-9-fluoroandrosta-1,4-diene-3-one (1.1 g) is suspended in dry diethylbenzene (30 ml). After refluxing for twenty minutes, the solution is cooled, poured on a column of silica gel (15 g) and the column is eluted successively with chloroform-hexane (7:3), chloroform and chloroform-ethyl acetate (95:5) to isolate the title compound (900 mg) and 11β-acetyloxy-9-fluoroandrosta-1,4-diene-3,17-dione (120 mg). Crystallization of the 900 mg of material from ethyl acetate-hexane gives 800 mg of material, melting point 192—194°C, with consistent spectral data.

D) 11β-Acetyloxy-17-(ethylthio)-9-fluoro-17-(methylthio)androsta-1,4-diene-3-one

To a solution of 11β-acetyloxy-9-fluoro-17-(methylthio)androsta-1,4-diene-3-one (632 mg) in dry dichloromethane (20 ml) containing dry ethanethiol (0.6 ml) is added boron trifluoride etherate (0.25 ml). After one hour, the mixture is added to a $NaHCO_3$ solution and extracted with chloroform. The chloroform

solution is washed with water, dried ($MgSO_4$ anhydrous), and evaporated to afford the title compound (620 mg) contaminated with a small amount of 11β-acetyloxy-9-fluoroandrosta-1,4-diene-3,17-dione. This material is used in the next step without further purification. A specimen crystallized from acetone-hexane shows melting point 140—142°C and a consistent nmr spectrum.

E) 17-(Ethylthio)-9-fluoro-11β-hydroxy-17-(methylthio)androsta-1,4-diene-3-one

A solution of 11β-acetyloxy-17-(ethylthio)-9-fluoro-17-(methylthio)androsta-1,4-diene-3-one (620 mg) in a mixture of methanol (20 ml) and tetrahydrofuran (10 ml) is stirred under nitrogen with 3M aqueous sodium hydroxide (1.5 ml). After eighteen hours, a slight excess of acetic acid is added. The mixture is concentrated *in vacuo*, diluted with water and extracted with chloroform. The chloroform solution is washed with water, dried, evaporated and chromatographed over a column of silica gel (10 g) eluting the column successively with chloroform-hexane (4:1), chloroform and chloroform-ethyl acetate (95:5) to isolate the title compound (550 mg). Crystallization of this from ethyl acetate-hexane and drying (110°C, 0.4 mbar (0.3 mm of Hg), 7 hours) gives the analytical specimen, melting point 275°C, *dec.* (contracts from approximately 200°C, discoloration starts from approximately 220°C and becomes deeper until spontaneous melting at 275°C with decomposition) with consistent spectral data. The nmr spectrum shows that this is an essentially 1:1 mixture of the two 17-stereoisomers.

| | | | | |
|---|---|---|---|---|
| Anal. Calcd. for $C_{22}H_{31}FO_2S_2$: | C, 64.35; | H, 7.61; | F, 4 63 | S, 15.62 |
| Found: | C, 64.16; | H, 7.69; | F, 4.59; | S, 15.49 |

Example 11

17-(Ethylthio)-9-fluoro-11β-hydroxy-17-(phenylthio)androsta-1,4-dien-3-one, isomer A

A) 11β-Acetyloxy-9-fluoroandrosta-1,4-diene-3,17-dione

A solution of 20 g of 9-fluoro-11β-hydroxyandrosta-1,4-diene-3,17-dione, 120 ml of glacial acetic acid, 120 ml of acetic anhydride and 5 g of *p*-toluenesulfonic acid is stirred at room temperature under nitrogen for 24 hours. The resulting solution is quenched with 5 g of sodium acetate. The solvent is partially removed *in vacuo* at 35—40°C and the resultant slurry is diluted with chloroform. The chloroform solution is washed with water, saturated sodium bicarbonate and water, dried over anhydrous $Na_2SO_4$ and evaporated *in vacuo* to give the title compound. This is crystallized from ethyl acetate-hexane to give 20 g of the title compound melting point 171—174°C, with consistent spectral data.

B) 11β-Acetyloxy-17,17-bis(ethylthio)-9-fluoroandrosta-1,4-dien-3-one

A solution of 20 g (55.5 mmole) of 11β-acetyloxy-9-fluoroandrosta-1,4-diene-3,17-dione in 75 ml of dry dichloromethane and 75 ml of glacial acetic acid is stirred with 10 ml of ethanethiol and 2 ml of boron trifluoride etherate at room temperature under nitrogen. After 2.0 hours the resulting solution is diluted with dichloromethane, washed with water, saturated sodium bicarbonate and water, dried over anhydrous $Na_2SO_4$ and evaporated *in vacuo*. The gummy residue is dissolved in 1:1 chloroform-hexane and chromatographed on a 150 g-silica gel column, eluting successively with chloroform-hexane (1:1, 6:4 and 7:3), chloroform, chloroform-ethyl acetate (5:95 and 1:9) and methanol-chloroform (1:9) to give 9.5 g of unreacted 11β-acetyloxy-9-fluoroandrosta-1,4-diene-3,17-dione, 8.2 g of overreacted steroidal product and 5.2 g of the title compound, melting point 246—250°C, with consistent spectral data.

C) 11β-Acetyloxy-17-(ethylthio)-9-fluoroandrosta-1,4,16-trien-3-one

A suspension of 5.2 g of 11β-acetyloxy-17,17-bis(ethylthio)-9-fluoroandrosta-1,4-dien-3-one in 85 ml of dry diethylbenzene is stirred at 180°C (oil bath temperature) for 1.0 hour; the suspension gradually becomes a homogeneous solution during the heating. The resulting solution is cooled to 0°C and the solid that precipitates is filtered and dried *in vacuo* to give 3.6 g of the title compound, melting point 211—215°C, with consistent spectral data.

The filtrate is chromatographed on a 30 g-silica gel column, eluting successively with chloroform-hexane (1:1) and chloroform to give 0.4 g more of the title compound.

D) 11β-Acetyloxy-17-(ethylthio)-9-fluoro-17-(phenylthio)androsta-1,4-dien-3-one

A solution of 1.2 g (2.97 mmole) of 11β-acetyloxy-17-(ethylthio)-9-fluoroandrosta-1,4,16-trien-3-one, 18 ml of dry dichloromethane and 0.8 ml of thiophenol is cooled to −20°C under nitrogen. Boron trifluoride etherate (0.6 ml) is then added. The solution is stirred at −10° to −20°C for 2 hours under nitrogen. The resulting solution is diluted with dichloromethane, washed with saturated sodium bicarbonate and water, dried over anhydrous $Na_2SO_4$ and evaporated *in vacuo* to give a gum. This is dissolved in chloroform-hexane (1:1) and chromatographed on a 20 g-silica gel column, eluting successively with chloroform-hexane (1:1), chloroform, chloroform-ethyl acetate (95:5) and chloroform-methanol (9:1) to give 1.0 g of impure title compound. This is dissolved in the minimum amount of ethyl acetate and stood at room temperature for 2 days. The solid that precipitates is filtered to give 480 mg of title compound, melting point 152—158°C, with consistent spectral data.

E) 17-(Ethylthio)-9-fluoro-11β-hydroxy-17-(phenylthio)androsta-1,4-dien-3-one, isomer A

A stream of nitrogen is bubbled through a solution of 560 mg of 11β-acetyloxy-17-(ethylthio)-9-fluoro-17-(phenylthio)androsta-1,4-dien-3-one in a mixture of tetrahydrofuran (30 ml), methanol (15 ml) and water (1.0 ml) for 15 minutes; sodium hydroxide (12%, 1.2 ml) is then added. The solution is stirred at room temperature under nitrogen for 1.5 hour, acidified with acetic acid, and evaporated *in vacuo* to give a solid. This is rinsed with a small amount of water to give 480 mg of the title compound. Recrystallization from acetone-hexane gives 400 mg of an analytical specimen, melting point 272—273°C *dec.*, with consistent spectral data.

| | | | | |
|---|---|---|---|---|
| Anal. Calcd. for $C_{27}H_{33}FO_2S_2$: | C, 68.61; | H, 7.04; | F, 4.02; | S, 13.57 |
| Found: | C, 68.37; | H, 7.00; | F, 4.08; | S, 13.56 |

Example 12

17-(Ethylthio)-9-fluoro-11β-hydroxy-17-(phenylthio)androsta-1,4-dien-3-one, isomer B

A) 11β-Acetyloxy-17,17-bis(phenylthio)-9-fluoroandrosta-1,4-dien-3-one

A solution of 8.5 g of 11β-acetyloxy-9-fluoroandrosta-1,4-diene-3,17-dione (see example 10A) in 60 ml of dry dichloromethane and 60 ml of glacial acetic acid is stirred with 8.0 ml of thiophenol and 1.5 ml of boron trifluoride etherate at room temperature under nitrogen for 3.5 hours. The resulting solution is diluted with dichloromethane, washed with water, saturated sodium bicarbonate and water, dried over anhydrous $Na_2SO_4$ and evaporated *in vacuo*. The gummy residue is dissolved in 7:3 chloroform-hexane and chromatographed on a 100 g silica gel column, eluting successively with chloroform-hexane (7:3), chloroform, chloroform-ethyl acetate (9:1) and chloroform-methanol (9:1) to give 4.8 g of unreacted 11β-acetyloxy-9-fluoroandrosta-1,4-diene-3,17-dione, 3.0 g of over-reacted steroidal product and 1.5 g of the title compound, melting point 233—235°C, with consistent spectral data.

B) 11β-Acetyloxy-9-fluoro-17-(phenylthio)androsta-1,4,16-trien-3-one

A suspension of 1.5 g of 11β-acetyloxy-17,17-bis(phenylthio)-9-fluoroandrosta-1,4-dien-3-one in 25 ml of dry diethylbenzene is stirred at 185—190°C (oil bath temperature) for 1.0 hour. The suspension gradually becomes a homogeneous solution during the heating and the resulting solution is cooled to 0°C. The solid that precipitates is filtered and dried *in vacuo* to give 0.9 g of the title compound, melting point 228—229°C, with consistent spectral data.

The filtrate is chromatographed on a 25 g-silica gel column, eluting successively with 1:1 chloroform-hexane and 1:9 chloroform-ethyl acetate to give 0.25 g more of the title compound.

C) 11β-Acetyloxy-17-(ethylthio)-9-fluoro-17-(phenylthio)androsta-1,4-dien-3-one, Isomer B

A solution of 1.0 g of 11β-acetyloxy-9-fluoro-17-(phenylthio)androsta-1,4,16-trien-3-one, 12 ml of dry dichloromethane and 0.5 ml of ethanethiol is cooled to −10°C under nitrogen. Boron trifluoride etherate is then added. The solution is stirred at −10°C under nitrogen for 2.5 hours. The resulting solution is diluted with dichloromethane, washed with saturated sodium bicarbonate and water, dried over anhydrous $Na_2SO_4$ and evaporated *in vacuo* to give a foamy solid. This is dissolved in chloroform and chromatographed on two precoated silica gel TLC plates (E. Merck, 20 cm × 20 cm × 2 mm, 1:4 ethyl acetate-chloroform for development) to give 800 mg of slightly impure title compound. This is crystallized from ethyl acetate-hexane to give 500 mg of title compound, melting point 145—148°C, with consistent spectral data.

D) 17-(Ethylthio)-9-fluoro-11β-hydroxy-17-(phenylthio)androsta-1,4-dien-3-one, isomer B

A stream of nitrogen is bubbled through a solution of 545 mg of 11β-acetyloxy-17-(ethylthio)-9-fluoro-17-(phenylthio)androsta-1,4-dien-3-one in a mixture of tetrahydrofuran (30 ml), methanol (15 ml) and water (1.0 ml) for 15 minutes. Sodium hydroxide (12%, 1.2 ml) is then added. The solution is stirred at room temperature under nitrogen for 1.5 hours. The resulting solution is acidified with acetic acid and the solvent is evaporated *in vacuo* to give a solid. This is rinsed with a small amount of water and filtered. The solid is recrystallized from acetone-hexane to give 370 mg of an analytical specimen, melting point 263—264°C, *dec.*, with consistent spectral data.

| | | | | |
|---|---|---|---|---|
| Anal. Calcd. for $C_{27}H_{33}FO_2S_2$: | C, 68.61; | H, 7.04; | F, 4.02; | S, 13.57 |
| Found: | C, 68.73; | H, 6.81; | F, 4.04; | S, 13.52 |

Example 13

17-(Butylthio)-17-(ethylthio)-9-fluoro-11β-hydroxyandrosta-1,4-dien-3-one

A) 11β-Acetyloxy-17-(butylthio)-17-(ethylthio)-9-fluoroandrosta-1,4-diene-3-one

A solution of 11β-acetyloxy-17-(ethylthio)-9-fluoroandrosta-1,4,16-triene-3-one (700 mg; see example 11c) and n-butanethiol (271 mg) is cooled and stirred in a bath at −40 to −45°C and distilled boron trifluoride etherate (0.3 ml) is added. After 2.0 hours at −40 to −45°C, the solution is gradually warmed to 10°C in the course of 1.5 hours. The mixture is then diluted with dichloromethane, washed successively with a saturated sodium bicarbonate solution and brine, dried ($MgSO_4$ anh.) and is evaporated to afford the crude title compound. This material is chromatographed over a column of silica gel (20 g), eluting the column with chloroform-hexane mixtures (1:1, 1:4), chloroform and chloroform-ethyl acetate (95:5) to afford the title compound (700 mg). Examination of the nmr spectrum and tlc behavior shows that this material is contaminated with a significant amount of the starting steroid which is not readily separable

from the more polar isomer of the product under the tlc systems examined. Partial separation of the two 17-isomers is achieved under these tlc conditions.

B) 17-(Butylthio)-17-(ethylthio)-9-fluoro-11β-hydroxyandrosta-1,4-dien-3-one

11β-Acetyloxy-17-(butylthio)-17-(ethylthio)-9-fluoroandrosta-1,4-diene-3-one (925 mg) is dissolved in a mixture of methanol (20 ml) and tetrahydrofuran (20 ml). The solution is stirred, purged with nitrogen and 3.0 M sodium hydroxide (3.0 ml) is added. After 2 hours a slight excess of acetic acid is added and the mixture is then concentrated *in vacuo*. It is diluted with water (150 ml) and extracted with chloroform. The chloroform extract is washed with water, dried ($MgSO_4$ anh.) and evaporated to afford the title compound (870 mg). Examinations of the nmr spectrum and tlc behavior of this compound show the presence of some 17-(ethylthio)-9-fluoro-11β-hydroxyandrosta-1,4,16-triene-3-one and two 17-stereoisomers of the title compound. The material is applied on four 2.0 mm Merck silica gel plates and the plates are developed twice with chloroform-ethyl acetate (7:3). The products from the upper $\frac{1}{3}$ and lower $\frac{2}{3}$ of the broad band are isolated separately by extraction with chloroform-methanol (3:1) to afford respectively 340 and 527 mg of solids. One crystallization of the 340 mg of solid from ethyl acetate-hexane (1:1) and drying (100°C, 0.4 mbar (0.3 mm of Hg), 20 hours) gives the analytical specimen of the title compound (2881 mg) melting point 118—125°C with consistent spectral data.

Anal. Calcd. for $C_{25}H_{37}FO_2S_2$: C, 66.33; H, 8.24; F, 4.20; S, 14.16
Found: C, 66.10; H, 8.19; F, 4.01; S, 13.98

Example 14

17α-(Ethylthio)-9-fluoro-11β-hydroxy-17-(methylthio)androsta-1,4-dien-3-one

A) 11β-Acetyloxy-17α-(ethylthio)-9-fluoro-11-hydroxy-17-(methylthio) androsta-1,4-diene-3-one

A solution of 11β-acetyloxy-9-fluoro-17-(methylthio)androsta-1,4,16-triene-3-one (2.1 g), in dry dichloromethane (45 ml) containing dry ethanethiol (1.5 ml) is cooled in a bath at about −40°C (acetonitrile-dry ice bath) and boron trifluoride etherate (1.5 ml) is added. After 2.0 hours at about −40°C the reaction is quenched by the addition of a 10% sodium carbonate solution under vigorous stirring at the low temperature. The mixture is then warmed to room temperature, diluted with water and extracted with chloroform. The chloroform extracts are combined, washed with water, dried ($MgSO_4$ anhydrous) and evaporated to afford the title compound in quantitative yield (2.38 g). Crystallization of this solid from acetone-hexane affords the analytical specimen (1.8 g), melting point 170—172°C with consistent spectral data.

B) 17α-(Ethylthio)-9-fluoro-11β-hydroxy-17-(methylthio)androsta-1,4-dien-3-one

A solution of 11β-Acetyloxy-17α-(ethylthio)-9-fluoro-17-(methylthio)androsta-1,4-diene-3-one (1.85 g) in a mixture of methanol (15 ml) and tetrahydrofuran (15 ml) is stirred with 3 M sodium hydroxide solution (2.5 ml) for 1.5 hours. A moderate excess of acetic acid is then added and the mixture is concentrated *in vacuo* to a slurry (about 10 ml). This is diluted with ice-cold water, the precipitated solid is isolated by filtration, washed with water and dried to afford the title compound (1.62 g). Crystallization of this from acetone-hexane gives the analytical specimen (1.25 g) melting point 218—220°C, with resolidification and remelting at 261—265°C with decomposition and discoloration.

Anal. Calc'd for $C_{22}H_{31}FO_2S_2$: C, 64.35; H, 7.61; F, 4.63; S, 15.62
Found: C, 64.57; H, 7.61; F, 4.85; S, 15.59

Example 15

17β-(Ethylthio)-9-fluoro-11β-hydroxy-17-(methylthio)androsta-1,4-dien-3-one

A) 11β-Acetyloxy-17β-ethylthio-9-fluoro-17-(methylthio)androsta-1,4-dien-3-one

A solution of 1.01 g (2.5 mmole) of 11β-acetyloxy-17-(ethylthio)-9-fluoroandrosta-1,4,16-trien-3-one in 15 ml of dry dichloromethane and 3.4 ml of a solution of methyl mercaptan in dichloromethane (1.82 g in 10 ml of dry dichloromethane) is cooled to about −40°C (acetonitrile-dry ice bath) under nitrogen; boron trifluoride etherate (0.7 ml) is then added. The solution is stirred at approximately −40°C under nitrogen for 3 hours, quenched with a saturated sodium bicarbonate solution at −40°C under vigorous stirring, diluted with chloroform, washed with water, dried over anhydrous $Na_2SO_4$ and evaporated *in vacuo* to give 1.0 g of the title compound, melting point 185—186°C, with consistent spectral data.

B) 17β-(Ethylthio)-9-fluoro-11β-hydroxy-17-(methylthio)androsta-1,4-dien-3-one

A stream of nitrogen is bubbled through a solution of 1.0 g of 11β-acetyloxy-17β-(ethylthio)-9-fluoro-17-(methylthio)androsta-1,4-dien-3-one in a mixture of tetrahydrofuran (30 ml), methanol (25 ml) and water (2 ml) for 15 minutes. Sodium hydroxide (12%, 2.5 ml) is then added. The solution is stirred at room temperature under nitrogen for 2 hours, acidified with acetic acid and evaporated *in vacuo* to give a slurry. This is redissolved in chloroform, washed with water, dried over anhydrous $Na_2SO_4$ and evaporated *in vacuo* to give the title compound (855 mg). Crystallization from acetone-hexane gives 710 mg of the analytical specimen, melting point 258—259°C dec. with consistent spectral data.

Anal. Calc'd for $C_{22}H_{31}FO_2S_2$: C, 64.35; H, 7.61; F, 4.63; S, 15.62
Found: C, 64.57; H, 7.42; F, 4.79; S, 15.65

Example 16

11β-Hydroxy-17,17-bis(methylthio)androsta-1,4-dien-3-one

Boron trifluoride etherate (3.0 ml) is added to a solution of 11β-hydroxyandrosta-1,4-diene-3,17-dione (6.5 g) in glacial acetic acid containing 3.0 ml of methanethiol. After 45 minutes, the solution is diluted with chloroform and added to water. The chloroform solution is washed with water, a saturated sodium bicarbonate solution and water, dried ($MgSO_4$ anh.) and evaporated to a gummy residue. This is chromatographed on a column of silica gel (50 g), eluting the column successively with chloroform-hexane, chloroform and chloroform-ethyl acetate mixtures (9:1 and 8:2) to afford successively the over-reacted steroid (~500 mg), the title compound (4.0 g) and unreacted starting steroid (2.0 g). One crystallization of the 4.0 g material from ethyl acetate-hexane and drying (75°C, 0.4 mbar (0.3 mm of Hg), 5.0 hours) affords the analytical specimen of the title compound as colorless crystals (3.6 g), melting point 203—204°C *dec.* with consistent spectral data.

| | | | |
|---|---|---|---|
| Anal. Calc'd for $C_{21}H_{30}O_2S_2$: | C, 66.61; | H, 7.99; | S, 16.90 |
| Found: | C, 66.72; | H, 7.96; | S, 17.50 |

Example 17

17α-(Ethylthio)-11β-hydroxy-17-(methylthio)androsta-1,4-dien-3-one

A) 11β-Hydroxy-17-(methylthio)androsta-1,4,16-triene-3-one

A suspension of 11β-hydroxy-17,17-bis(methylthio)androsta-1,4-diene-3-one (2.6 g) in dry diethylbenzene (120 ml) is refluxed for 1.0 hour in a bath at about 200°C. The resulting solution is then cooled to room temperature and subsequently in an ice bath to afford the title compound as needles (2.1 g), after filtration and washing with hexane. The filtrate is subsequently chromatographed on a column of silica gel (30 g) to afford another 100 mg of product. The total yield is thus 2.2 g of product, melting point 240—241°C, *dec.,* with a consistent nmr spectrum.

B) 17α-(Ethylthio)-11β-hydroxy-17-(methylthio)androsta-1,4-diene-3-one

A suspension of 11β-hydroxy-17-(methylthio)androsta-1,4,16-triene-3-one (1.0 g) in dichloromethane (70 ml) containing ethanethiol (1.12 g; 1.36 ml) is cooled and stirred in a bath at −78°C (acetone-dry ice) and boron trifluoride etherate (860 mg; 0.76 ml) is added. After 2.0 hours, the reaction is quenched by the addition of a solution of sodium hydroxide (2.0 g) in methanol-water (1:1; 30 ml) under vigorous stirring. The mixture is then warmed to room temperature, diluted with 20% hydrochloric acid (50 ml) and extracted with chloroform. The chloroform solution is washed with a dilute sodium bicarbonate solution and water, dried ($MgSO_4$ anhydrous), evaporated and the residue is chromatographed on a column of silica gel (20 g) eluting with chloroform and chloroform-ethyl acetate (9:1) to remove some 17-ketone that is present. The title compound obtained (1.03 g) is crystallized from ethyl acetate-hexane and dried (75°C, 0.4 mbar (0.3 mm of Hg), 7.0 hours) to afford the analytical specimen of the title compound (850 mg), melting point 176—178°C, with consistent spectral data.

| | | | |
|---|---|---|---|
| Anal. Calc'd for $C_{22}H_{32}O_2S_2$: | C, 67.30; | H, 8.22; | S, 16.33 |
| Found: | C, 67.54; | H, 7.92; | S, 16.26 |

Example 18

17β-(Ethylthio)-11β-hydroxy-17-(methylthio)androsta-1,4-dien-3-one

A) 17-(Ethylthio)-11β-hydroxyandrosta-1,4,16-trien-3-one

A suspension of 4.4 g of 17,17-bis(ethylthio)-11β-hydroxyandrosta-1,4-dien-3-one (see example 2) in 100 ml of dry diethyl benzene is stirred at 190—195°C (oil bath temperature) for 1.5 hours. The suspension becomes a solution during the heating; the resulting solution is cooled to 0°C. The solid that precipitates is filtered and dried *in vacuo* to give 3.0 g of the title compound, melting point 216—218°C, with consistent spectral data.

The filtrate is chromatographed on a 50-gram silica gel column, eluting successively with 1:1 chloroform-hexane and 9:1 chloroform-ethyl acetate to give 0.5 g more of the title compound.

B) 17β-(Ethylthio)-11β-hydroxy-17-(methylthio)androsta-1,4-dien-3-one

A suspension of 1.0 g of 17-(ethylthio)-11β-hydroxyandrosta-1,4,16-trien-3-one in 30 ml of dry dichloromethane and 1.5 ml of a solution of methyl mercaptan in dry dichloromethane (1.34 g in 10 ml of dry dichloromethane) is cooled to about −78°C (dry ice-acetone bath) under nitrogen. Boron trifluoride etherate (0.4 ml) is then added and the suspension gradually becomes a solution. The solution is stirred at −78°C under nitrogen for 5 hours, quenched with 5 ml of a solution of sodium hydroxide in methanol (2.0 g of sodium hydroxide in 40 ml of methanol) at about −70°C under vigorous stirring, diluted with chloroform and poured into water. The chloroform solution is separated, dried over anhydrous $Na_2SO_4$ and evaporated *in vacuo* to give 1.1 g of a foamy solid. A small scale run using 100 mg of 17-(ethylthio)-11β-hydroxyandrosta-1,4,16-trien-3-one gives 100 mg of material identical in tlc and NMR. These two are combined, dissolved in chloroform and chromatographed on a 25-gram silica gel column, eluting successively with chloroform and 5:95 ethyl acetate-chloroform to give 1.05 g of the title compound. Crystallization from acetone-hexane gives 850 mg of an analytical specimen, melting point 208—210°C, with consistent spectral data.

| | | | | |
|---|---|---|---|---|
| Anal. Calc'd for $C_{22}H_{32}O_2S_2$: | C, 67.30; | H, 8.22; | S, 16.33 |
| Found: | C, 67.42; | H, 8.36; | S, 16.34 |

Examples 19—24

Following the procedure of example 2, but substituting the steroid listed in column I for 11β-hydroxy-androsta-1,4-diene-3,17-dione and the thiol listed in column II for ethanethiol, yields the steroid listed in column III.

| | Column I | Column II | Column III |
|---|---|---|---|
| 19. | 9-Fluoro-11β-hydroxy-androsta-1,4-diene-3,17-dione | cyclohexanethiol | 17,17-bis(cyclohexylthio)-9-fluoro-11β-hydroxyandrosta-1,4-diene-3-one |
| 20. | 9-Fluoro-11β-hydroxy-androsta-1,4,6-triene-3,17-dione | ethanethiol | 17,17-bis(Ethylthio)-9-fluoro-11β-hydroxyandrosta-1,4,6-triene-3-one |
| 21. | 6α,9α-Difluoro-11β-hydroxyandrosta-1,4-diene-3,17-dione | methanethiol | 6α,9α-Difluoro-11β-hydroxy-17,17-bis(methylthio)androsta-1,4-diene-3-one |
| 22. | 9-Fluoro-11β-hydroxy 6α-methyl-androsta-1,4-diene-3,17-dione | propanethiol | 9-Fluoro-11β-hydroxy-6α-methyl-17,17-bis(propylthio)-androsta-1,4-diene-3-one |
| 23. | 9-Fluoro-11β,16α-dihydroxyandrosta-1,4-diene,3,17-dione | methanethiol | 9-Fluoro-11β,16α-dihydroxy-17,17-bis(methylthio)androsta-1,4-diene-3,17-dione |
| 24. | 9-Fluoro-11β-hydroxyandrosta-1,4-diene-3,17-dione | isobutylthiol | 9-Fluoro-11β-hydroxy-17,17 bis[(isobutyl)thio]androsta-1,4-diene-3,17-dione |

M.P. for Example 21 is 210°C—212°C, then resolidified and melts again at 225°C—227°C with decomposition.

Example 25

(11β,17α)-17-(Butylthio)-9-fluoro-11-hydroxy-17-(methylthio)androst-1,4-dien-3-one

A suspension of 696 mg (2 mmole) of 9-fluoro-11β-hydroxy-17-(methylthio)androsta-1,4,16-trien-3-one in 20 ml of dry dichloromethane and 2.14 ml (20 mmole) of 1-butanethiol is cooled to −78°C (acetone-Dry ice bath) under nitrogen. Boron trifluoride etherate (0.5 ml) is then added. The suspension gradually becomes a homogeneous solution. The solution is stirred at approximately −70°C under nitrogen for four hours, quenched with 4.5 ml of 5% sodium hydroxide in methanol at the low temperature under vigorous stirring, diluted with chloroform, washed with water, dried over anhydrous $Na_2SO_4$ and is evaporated *in vacuo* to give a foamy solid. This is dissolved in chloroform and chromatographed on 2 precoated silica gel TLC plates (20 cm × 20 cm × 2 mm, 1:4 ethyl acetate-chloroform for development) to isolate 460 mg (52.4%) of a tlc-homogeneous title compound. Crystallization from acetone-hexane gave 370 mg (42.2%) of an analytical specimen, m.p. 188—191°C, with consistent spectral data.

| | | | | |
|---|---|---|---|---|
| Anal. Calc'd for $C_{24}H_{35}FO_2S_2$: | C, 65.71; | H, 8.04; | F, 4.33; | S, 14.62 |
| Found: | C, 65.89; | H, 8.05; | F, 4.27; | S, 14.44 |

Example 26

(11β,17β)-9-Fluoro-11-hydroxy-17-(methylthio)-17-(propylthio)androst-1,4-dien-3-one

A) 11β-Acetyloxy-9-fluoro-17β-(methylthio)-17-(propylthio)androsta-1,4-dien-3-one

A solution of 500 mg (1.28 mmole) of 11β-acetyloxy-9-fluoro-17-(methylthio)-androsta-1,4,16-trien-3-one and 1.5 ml of 1-propanethiol in 15 ml of dry dichloromethane was cooled to −78°C (acetone-Dry ice bath) under nitrogen. Boron trifluoride etherate (0.32 ml) was then added. The solution was stirred at approximately −78°C for 6 hours, quenched with 2 ml of sodium hydroxide in methanol (2 g in 30 ml of methanol) at −78°C, diluted with chloroform, washed with water, dried over anhydrous $Na_2SO_4$ and evaporated in vacuo to give a foam. NMR spectrum indicated there was about 30—40% unreacted starting steroid. Using this as the starting material repeated the experiment as described above with same amounts of reagents under the same conditions to give 550 mg (92.1%) of a tlc-homogeneous title compound with consistent spectra data.

B) (11β,17β)-9-Fluoro-11-hydroxy-17-(methylthio)-17-(propylthio)androst-1,4-dien-3-one

A stream of nitrogen was bubbled through a solution of 550 mg (1.18 mmole) of 11β-acetyloxy-9-fluoro-17β-(methylthio)-17-(propylthio)androsta-1,4-dien-3-one in a mixture of tetrahydrofuran (30 ml), methanol (20 ml) and water (1.0 ml) for 15 minutes. Sodium hydroxide (12%, 1.2 ml) was then added. The solution was stirred at room temperature under nitrogen for 1.0 hour, acidified with acetic acid and evaporated *in vacuo* to give a slurry. This was diluted with chloroform, washed with water, dried over anhydrous $Na_2SO_4$ and evaporated *in vacuo* to give a slightly impure title compound. This was dissolved in 9:1 chloroform-hexane and chromatographed on a 15 g-silica gel column eluting with 9:1 chloroform-hexane and chloroform to give 460 mg (91.8%) of a tlc-homogeneous material. Crystallization fom ethyl acetate-hexane gave 380 mg (75.8%) of an analytical specimen, m.p. 194—197°C, with consistent spectra data.

| | | | | |
|---|---|---|---|---|
| Anal. Calc'd for $C_{23}H_{33}FO_2S_2$: | C, 65.05; | H, 7.83; | F, 4.47; | S, 15.10 |
| Found: | C, 64.91; | H, 7.63; | F, 4.47; | S, 15.05 |

Example 27

9-Fluoro-17,17-bis(methylthio)androsta-1,4-dien-3,11-dione

A solution of 500 mg (1.26 mmole) of 9-fluoro-11β-hydroxy-17,17-bis-(methylthio)androsta-1,4-dien-3-one in a mixture of dimethyl sulfoxide (6 ml), acetic anhydride (4 ml) and glacial acetic acid (2 ml) was stirred at room temperature under nitrogen for 3 days. The resulting solution was slowly poured into a cold saturated sodium bicarbonate solution and extracted with chloroform. The chloroform solution was washed with water, dried over anhydrous $Na_2SO_4$ and evaporated *in vacuo* to give a foam. This was dissolved in chloroform and chromatographed on a 15 gram silica gel column eluting with chloroform and 4:1 chloroform-methanol to give 380 mg (76.2%) of the title compound. The title compound (380 mg) was recrystallized from acetone-hexane to give 300 mg (60.2%) of an analytical specimen, m.p. 197—199°C, with consistent spectral data.

| | | | | | |
|---|---|---|---|---|---|
| Anal. for $C_{21}H_{27}FO_2S_2$: | Calc'd: | C, 63.92; | H, 6.90; | F, 4.82; | S, 16.25 |
| Found: | | C, 63.76; | H, 7.08; | F, 4.94; | S, 16.08 |

Example 28

(11β)-17,17-Bis((cyclopropylmethyl)thio)-9-fluoro-11-hydroxyandrosta-1,4-dien-3-one

To a mechanically stirred solution of 9-fluoro-11β-hydroxyandrosta-1,4-diene 3,17-dione (1.95 g) in 15.0 ml of dry dichloromethane and 15.0 ml of glacial acetic acid at −15—20°C and under nitrogen was injected 2.00 ml of cyclopropylmethyl mercaptan followed by 0.42 ml of boron trifluoride etherate. The reaction mixture turned yellow with time and after 70.0 minutes the reaction was quenched with a 100 ml solution of saturated sodium carbonate. The mixture was extracted with chloroform (four washings at 200 ml/wash) and the collected chloroform layer was washed with 800 ml of water. The chloroform layer was then dried over anhydrous $MgSO_4$, filtered and rotovaped to dryness. The residue was dissolved in (7:3) chloroform-hexane and added to a medium sized gravity column containing 50 g of Baker silica gel (250 μm—75 μm) [60—200 mesh]. Chloroform neat was used as the mobile phase to remove the overreacted material and then a 5% ethyl acetate:chloroform 95% mobile phase was used to elute the clean title compound which was rotovaped to dryness and recrystallized from ethyl acetate followed by drying (65°C, 20 mbar (15 mm Hg), 12.0 hours) to yield the analytical specimen (85.0 mg, 2.9%) with consistent spectral data. m.p. 173—175°C.

| | | | | |
|---|---|---|---|---|
| Anal. Calc'd for $C_{27}H_{37}O_2S_2F$: | C, 68.03; | H, 7.82; | S, 13.45; | F, 3.99 |
| Found: | C, 68.20; | H, 7.85; | S, 13.21; | F, 3.96 |

Example 29

11β-Acetyloxy-17α-(ethylthio)-6,9-difluoro-17β-(methylthio)androsta-1,4-dien-3-one

A) 11β-acetyloxy-6α,9-difluoro-17,17-bis(methylthio)androsta-1,4-diene-3-one

4.0 g of 6α,9α-Difluoro-11β-hydroxy-17,17-bis(methylthio)androsta-1,4-diene-3-one was refluxed at 105°C under nitrogen in pyridine (120.0 ml) and acetic anhydride (60.0 ml) for 19.0 hours. With time the mixture became dark brown and after 19.0 hours the mixture was cooled and rotovaped to yield a brown syrup. This was dissolved in 200 ml of a 10% HCl solution and four chloroform washings were performed (200 ml/wash), the chloroform extracts were pooled, washed with 800 ml of water, dried over anh. $MgSO_4$, filtered and rotovaped to dryness in preparation for a column. Two gravity columns were run (50 g Baker silica gel 250 μm—75 μm (60—200 mesh), 5%:95% ethyl acetate:chloroform used as mobile phase to remove desired product) and the fractions containing product were pooled and rotovaped to a foam which was recrystallized from ethyl acetate and hexane. NMR and TLC (7:3 chloroform:ethyl acetate) were in agreement with the title compound. The crystals were dried (40°C, 20 mbar (15 mm Hg), 6.0 hours) and weighed (1.3 g, 29.5%).

B) 11β-acetyloxy-6α,9-difluoro,17-methylthio-androsta-1,4,16,triene,3-one

1.2 g of compound A was refluxed for 1.5 hours at 195°C in diethylbenzene. Upon cooling brownish crystals precipitated which were filtered and dried (50°, 20 mbar (15 mm Hg), 12.0 hours). The filtrate was added to a 15 g Baker silica gel gravity column to remove the diethylbenzene. A 5% ethyl acetate:95%

chloroform mobile phase eluted the desired product. NMR, HPLC, and TLC (7:3 chloroform:ethyl acetate) all were in agreement with the pyrolysis product. Total yield was 1.062 g, 99%.

C) 11β-acetyloxy-17α-ethylthio-6α,9-difluoro-17β-methylthio androsta-1,4-dien-3-one

817 mg (2.0 mmole) of compound B was dissolved in 30.0 ml of dry dichloromethane with mechanical stirring and a nitrogen atmosphere. To the homogeneous mixture maintained at −78°C via a dry ice-acetone bath was injected ethyl mercaptan (1.33 ml., 18.0 mmole) followed by boron trifluoride etherate (1.13 ml, 9.0 mmole). An intense yellow color ensued with time and after 9.0 hours the reaction was quenched in 100 ml of a (1:1) water:methanol solution at −78°C. The mixture was then allowed to warm to room temperature before it was extracted with chloroform (four washings, 200 ml/wash), the chloroform layers pooled, washed with 800 ml of water, dried over anh. $MgSO_4$ and rotovaped to yield a brownish oil. Analytical HPLC (c=1 mg/ml $CH_3CN$, $C_{18}$ μ-Bondapak (column) revealed the presence of six compounds whereas TLC (7:3 chloroform:ethyl acetate) showed only a single spot. HPLC results showed the following:

| Retention time in min. | Interpretation of Peak |
|---|---|
| 1) 2.8 | starting material (17-ketone) (minor) |
| 2) 4.0 | 17-ketone impurities (minor) |
| 3) 4.9 | 17-ketone impurities (minor) |
| 4) 14.9 | vinyl. sulfide (minor) |
| 5) 19.8 | ethyl, methyl (desired product) (major) |
| 6) 27.2 | diethyl (major) |

A successful separation was obtained using preparative HPLC (Water's 500; reverse phase $C_{18}$ μ-Bondapak column; mobile phase, 55% acetonitrile 45% 0.05 M sodium dihydrogen phosphate; flow rate, 250 ml/60 sec. 600 mg of the mixture was added to the column and 38 fractions were collected in all with fractions 25—29 being pooled as containing clean desired product. These fractions were rotovaped to a few ml volume, and crystallization occurred upon cooling. The crystals were filtered, washed with cold water, dried (20 mbar [15 mm Hg], 80°C 12 hours) and weighed (50 mg). Analytical HPLC of the material showed a single peak at 19.8 min corresponding to the desired product.

D) 11β-acetyloxy-17α-(ethylthio)-6,9-difluoro-17β-(methylthio)androsta-1,4-dien-3-one

50.0 mg of compound C was dissolved in 4.0 ml of a (1:1) methanol:THF solution with magnetic stirring and a nitrogen atmosphere. 0.1 ml of a 3.0 M sodium hydroxide solution was then injected turning the mixture a pale yellow. After 1.5 hours at room temperature the reaction mixture was extracted with chloroform (3 washings, 50 ml/wash). The chloroform layer was washed with 150 ml water, separated, dried over anh. $MgSO_4$, filtered, and rotovaped to a solid which was recrystallized from ethyl acetate to yield the analytical specimen (35 mg, 77%) which was dried (20 mbar [15 mm Hg] 100°C, 12.0 hours) and found to have consistent spectral data.

| | | | | | |
|---|---|---|---|---|---|
| Anal.for $C_{22}H_{30}O_2S_2F_2$: | Calc'd: | C, 61.65; | H, 7.05; | S, 14.96; | F, 8.87. |
| Found: | | C, 61.77; | H, 7.11; | S, 14.73; | F, 8.61. |

Melting point — 176—180°C.

Example 30

(11β)-17,17-Bis((isopropyl)thio)-9-fluoro-11-hydroxyandrosta-1,4-dien-3-one

To a magnetically stirred solution of 9-fluoro-11β-hydroxyandrosta-1,4-diene-3,17-dione (1.87 g) in 9.0 ml of dry dichloromethane and 21.0 ml of glacial acetic acid under nitrogen and maintained at −10°C (dry ice/acetone bath) was added 10.0 ml of distilled isopropyl mercaptan followed by 1.47 ml of boron trifluoride etherate. The reaction mixture turned bright yellow after $BF_3$:$Et_2O$ injection and a precipitate was observed after 30.0 minutes of reaction time. The reaction was quenched after 75.0 minutes with a saturated sodium carbonate solution followed by extraction with chloroform. The chloroform layer was then dried over anhydrous magnesium sulfate, filtered, and rotovaped *in vacuo* to a solid. The solid residue was dissolved in (7:3) chloroform-hexane, preadsorbed on Baker silica gel (250 μm—75 μm) [60—200 mesh] and flash chromatographed. A (5:95) ethyl acetate-chloroform mobile phase was used to elute the desired product which was rotovaped *in vacuo* to dryness and recrystallized from ethyl acetate-hexane. The white crystals (.58 g, 22.0%) were dried (100°C, 20 mbar (15 mm Hg), 12.0 hours) and the spectral data was consistent with the assigned structure.

Anal.Calc'd for $C_{25}H_{37}O_2S_2F_1$: C, 66.33; H, 8.24; S, 14.17; F, 4.20.
Found: C, 66.33; H, 7.99; S, 14.00; F, 4.24.
Melting point — 245—249°C.

Example 31

(11β,17α)-17-(cyclohexylthio)-9-fluoro-11-hydroxy-17-(methylthio)androsta-1,4-dien-3-one

A) 11β-Acetyloxy-17α(cyclohexylthio)-9-fluoro-17-methylthio androsta-1,4-diene-3-one

To a magnetically stirred solution under a nitrogen atmosphere containing 11β-acetyloxy-9-fluoro-17-methylthio, androsta-1,4-diene-3-one (1.13 g, 3.0 mmole) dissolved in dry, distilled dichloromethane (45.0 ml) at −78°C (dry ice/acetone) was added cyclohexyl mercaptan (1.83 ml, 15.0 mmole) followed by boron trifluoride etherate (1.0 ml, 8.0 mmole). A sample was removed and monitored on analytical HPLC ($C_{18}$ μ-Bondapak) after 4 hours and 20 minutes of reaction time. Some starting material was still present so the reaction was allowed to continue for another 3 hours and 40 minutes before quenching it with a saturated $Na_2CO_3$ solution at 0°C. The mixture was allowed to warm and was then chloroform extracted, washed with water, dried over anhydrous $MgSO_4$ and rotovaped to a yellow oil (1.0 g, 68.2%). HPLC analysis showed only one major peak with a retention time of 44.61 minutes.

Preparation of (11β,17α)-17-(cyclohexylthio)-9-fluoro-11-hydroxy-17-(methylthio)androsta-1,4-dien-3-one

Compound A (1.0 g) was dissolved in dry tetrahydrofuran (48.0 ml), methanol (24.0 ml), and water (6.0 ml) with magnetic stirring and under a nitrogen atmosphere. A 3 N sodium hydroxide solution (3.0 ml) was added dropwise causing the mixture to turn an orange brown color. A TLC check after 105 minutes revealed the starting material had been completely converted to product. After 120 minutes total reaction time the mixture was chloroform extracted (3 × 150 ml). The chloroform extracts were pooled, washed with water, dried over anhydrous $MgSO_4$ and rotovaped *in vacuo* to a yellow solid. This was dissolved in 30.0 ml of boiling ethyl acetate and put in the freezer to crystallize overnight. Colony-like white crystals formed along with a gel-like substance. The crystals were filtered and rinsed with cold ethyl acetate to yield the analytical specimen (0.614 g, 67.0%; dried 48.0 hours, 75°C, HV) of the title compound with consistent spectral data.

*Anal.* Calc'd for $C_{25}H_{37}O_2S_2F_1$: C, 67.20; H, 8.03; S, 13.80; F, 4.09.
Found: C, 66.92; H, 7.93; S, 13.50; F, 4.12.
Melting point — 288—290°C.

Example 32

6β,9-Difluoro-11β-hydroxy-17,17-bis(methylthio)androsta-1,4-dien-3-one

A solution of 6 α,9-difluoro-11β-hydroxy-androsta-1,4-diene-3,17-dione (1.43 g, 4.3 mmole) in a mixture of glacial acetic acid (20 ml) and $CH_2Cl_2$ (6 ml) was cooled and stirred in an acetone-ice bath at −10°C. Distilled $BF_3$:$Et_2O$ (0.6 ml) was then added. A deep yellow color developed. After 30 minutes, the solution was poured into water and was extracted with chloroform (3 × 50 ml). The extracts were combined, washed with water, a dilute $NaHCO_3$ solution and water, dried ($MgSO_4$ anh.) and was evaporated to afford the crude product. A tlc examination of this showed a significant amount of the starting material and 3 less polar products; the least polar of this was material where both A and D had reacted. The crude product was subjected to a chromatography on a column of silica gel (20 g) using $CHCl_3$-hexane mixtures, $CHCl_3$ and $CHCl_3$-EtOAc mixtures for elution to afford the title compound (600 mg, 33.7%) which was contaminated with a slightly more polar compound by tlc. It was therefore subjected to a preparative tlc on four 2.0 × 200 × 200 mm silica gel plates (Uniplates) using $CHCl_3$—EtOAc (9:1) for development to isolate the title compound (350 mg, 19.7%). AN HPLC assay[1] showed three impurities amounting to a total of ~15%. Two recrystallizations of this material successively from EtOAc-hexane and $CH_3OH$—$CH_2Cl_2$ gave the analytical specimen (120 mg, 6.7%) m.p. 182—184°C (dec.) with consistent spectral data. This specimen still had 5—7% of unknown impurities by HPLC assay but no effort was made to purify it any further.

Anal.Calc'd for $C_{21}H_{28}F_2O_2S_2$: C, 60.84; H, 6.81; F, 9.17; S, 15.47.
Found: C, 61.02; H, 6.88; F, 9.24; S, 16.66.
Melting point — 182—184°C.

Example 33

(11β)-17,17-Bis((n-dodecyl)thio)-9-fluoro-11-hydroxyandrosta-1,4-dien-3-one

To a stirred solution of 9-fluoro-11β-hydroxyandrosta-1,4-diene-3,17-dione (3.18 g, 10.0 mmole) dissolved in glacial acetic acid (60.00 ml) and distilled dichloromethane and maintained at −10°C (dry ice/acetone) in a dry, nitrogen purged flask was added n-dodecyl mercaptan (5.06 ml.) followed by 0.62 ml of boron trifluoride etherate. After a 15.0 minute period TLC showed no reaction so .62 ml more of boron trifluoride etherate was added. The reaction then proceeded slowly so .62 ml more of $BF_3$:$Et_2O$ and 2.5 ml more of the n-dodecyl mercaptan were added. After a total of 75.0 minutes the reaction mixture was quenched with cold water and then chloroform extracted. The pooled chloroform layers were washed with a saturated sodium carbonate solution, then dried over anhydrous magnesium sulfate, and rotovaped to a white slurry. The crude material was flash chromatographed by preadsorbing the material on Baker silica gel and then eluting the clean title compound with a 1% ethyl acetate:99% $CHCl_3$ mobile phase. The

compound was recrystallized from ethyl acetate to yield 150 mg (2.13%) of an analytical specimen with consistent spectral data.

Anal.Calc'd for $C_{43}H_{73}O_2S_2F_1$: C, 73.24; H, 10.43; S, 9.10; F, 2.70.
Found: C, 72.96; H, 10.33; S, 9.06; F, 2.64.
Melting point — 106—108°C.

### Example 34

(11β,16α)-16-Acetyloxy)-9-fluoro-11-hydroxy-17,17-bis(methylthio)androsta-1,4-diene-3-one

A solution of 200 mg (0.485 mmole) of (11β,16α)-9-fluoro-11,16-dihydroxy-17,17-bis(methylthio)androsta-1,4-dien-3-one and 2.5 ml of acetic anhydride in 15 ml of pyridine was stirred at room temperature under nitrogen overnight. The resulting solution was poured into a cold 5% hydrochloric acid solution and extracted with dichloromethane. The dichloromethane solution was washed with water, dried over anhydrous $Na_2SO_4$ and evaporated *in vacuo* to give a foam. This was chromatographed on 2 precoated silica gel TLC plates (E. Merck, 20 cm × 20 cm × 0.5 mm, 1:9 methanol-dichloromethane for development) to give 190 mg (86.2%) of tlc-homogeneous title compound. Crystallization from acetone-hexane gave 140 mg (63.5%) of an analytical specimen, m.p. 255—256°C, with consistent spectral data.

Anal.Calc'd for $C_{23}H_{31}FO_4S_2$: C, 60.76; H, 6.87; F, 4.18; S, 14.11
Found: C, 60.62; H, 6.87; F, 4.17; S, 14.20

### Example 35

(11β,17α)-9-Fluoro-11-hydroxy-17-((1-methylethyl)thio)-17-(methylthio)androsta-1,4-dien-3-one

To a stirred solution of 2.26 g 11α-acetyloxy-9-fluoro-17-methylthio, androsta-1,4-dien-3-one in 90 ml of dry $CH_2Cl_2$ maintained at −78°C there was added 3 ml of isopropylthiol followed by 2 ml of $BF_3{\cdot}Et_2O$ ($N_2$-atmosphere). The solution was kept at −78° for 7 hours, after which it was quenched with 5% NaOH. The product was extracted with 4 × 50 ml portions of EtOAc. Evaporation of the dried EtOAc extracts yielded an oil, which turned solid on treatment with EtOAc-Ether. Yield 1.5 g. HPLC ($C_{18}$ μ-Bondapak) 19.52 min. retention time, 94%.

To a solution of 0.8 g of the above acetate in 20 ml MeOH and 10 ml THF was added 10 ml of 3N NaOH ($N_2$-atmosphere). The mixture was stirred overnight, partly evaporated and the product precipitated with water. The resulting solid was filtrated off, washed with water and crystallized from EtOAc to yield 0.6 g of product, m.p. 285—287°C.

### Example 36

(11β)-9-Fluoro-11-hydroxy-17,17 bis(methylthio)androst-4-en-3-one

A) 3-Pyrrolidine Enamine of 9-fluoro-11-hydroxyandrost-4-en-3,17-dione:

10 g (0.0312 mole) of 9-fluoro-11-hydroxyandrost-4-en-3,17-dione was suspended in 300 ml of methanol, heated to almost reflux and then treated with pyrrolidine (4.0 ml). Immediate solution of the starting material occurred followed by the formation of light yellow precipitates within 10 minutes.

The reaction mixture was stirred for 1 hour at room temperature and the suspension concentrated down to a volume of 60 ml and filtered, washing the yellow compound with a small amount of methanol. Yield = 10.60 g, m.p. 252—254°C.

B) (11β)-9-Fluoro-11-hydroxy-17,17 bis(methylthio)androst-4-en-3-one

4.75 g (0.0127 mole) of the above enamine was dissolved in glacial acetic acid (95 ml) and methylene chloride (66.5 ml) and the resulting solution was cooled down to 0°C and treated with 35 ml of 2M $CH_3SH$ in $CH_2Cl_2$ and 4.75 ml of boron trifluoride etherate. The reaction mixture was stirred at 0—5° under nitrogen for 3 hours, poured into 1.0 liter of ice water, stirred for 15 minutes and then extracted with three 500-ml portions of dichloromethane. The organic extracts were combined, dried over anhydrous magnesium sulfate, filtered and the clear filtrate stripped to dryness. Yield: 9.9 g, syrup.

The crude thiol was dissolved in 95% ethanol (400 ml) and refluxed under nitrogen for 10 hours. The reaction mixture was cooled and stripped to dryness. Yield: 6.26 g. The crude product was dissolved in 800 ml of $CHCl_3$:Hexane (60:40), impregnated onto 90 g of silica gel and chromatographed by flash chromatography, eluting the column with 30 liters of $CHCl_3$:Hexane (60:40). The desired fractions were combined and stripped to dryness. Yield: 3.85 g, m.p. 215—216°C;

Anal.Calc'd for $C_{21}H_{31}FO_4S_2$: C, 63.28; H, 7.84; F, 4.77; S, 15.88.
Found: C, 63.35; H, 7.82; F, 4.65; S, 15.79.

**Claims for the Contracting States: BE CH DE FR GB IT LI LU NL SE**

1. A 3-ketoandrostene having in the 17-position the substituents $R_1$—S— and $R_2$—S— wherein $R_1$ and $R_2$ are different groups, and each is an alkyl, cycloalkyl or aryl group.

2. A 3-ketoandrostene in accordance with claim 1 having the formula

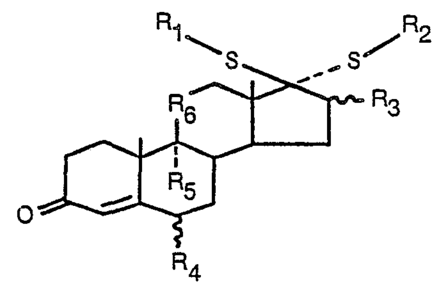

or 1,2-, 6,7- and 15,16-dehydro derivatives thereof, wherein

$R_1$ and $R_2$ are different $C_1$—$C_{12}$ alkyl, $C_{3-7}$ cycloalkyl, phenyl or phenyl substituted by one or two halogen atoms or one or two $C_{1-12}$ alkyl or alkoxy groups;

$R_3$ is hydrogen, hydroxy, alkoxy, aryloxy, alkylthio, arylthio,

$$\text{alkyl}-\overset{\overset{\displaystyle O}{\|}}{C}-O-$$

or halogen;

$R_4$ is hydrogen, methyl, hydroxy,

$$\text{alkyl}-\overset{\overset{\displaystyle O}{\|}}{C}-O-$$

or halogen;

$R_5$ is hydrogen or halogen; and

$R_6$ is carbonyl or β-hydroxymethylene.

3. A 3-ketoandrostene in accordance with claim 2 wherein $R_4$ is hydrogen.

4. A 3-ketoandrostene in accordance with claim 2 wherein $R_5$ is fluorine.

5. A 3-ketoandrostene in accordance with claim 2 wherein $R_6$ is β-hydroxymethylene.

6. A 3-ketoandrostene having the formula

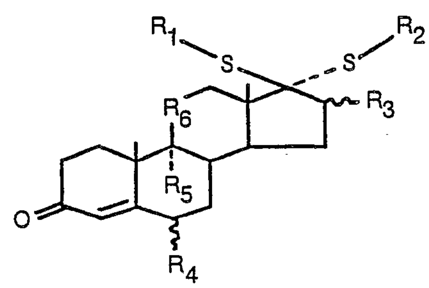

or 1,2-, 6,7- and 15,16-dehydro derivatives thereof, wherein

$R_1$ and $R_2$ are the same $C_{1-12}$ alkyl, $C_{3-7}$ cycloalkyl, phenyl or phenyl substituted by one or two hologen atoms or one or two $C_{1-12}$ alkyl or alkoxy groups;

$R_3$ is hydroxy, alkoxy, aryloxy, alkylthio, arylthio,

$$\text{alkyl}-\overset{\overset{\displaystyle O}{\|}}{C}-O-$$

or halogen;

$R_4$ is hydrogen, methyl, hydroxy,

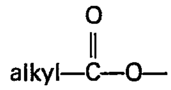

or halogen;

$R_5$ is hydrogen or halogen; and

$R_6$ is carbonyl or β-hydroxymethylene.

7. A 3-ketoandrostene in accordance with claim 6 wherein $R_4$ is hydrogen.

8. A 3-ketoandrostene in accordance with claim 6 wherein $R_5$ is fluorine.

9. A 3-ketoandrostene in accordance with claim 6 wherein $R_6$ is β-hydroxymethylene.

10. The 3-ketoandrostene in accordance with claim 6, (11β,16α)-17,17-bis(ethylthio)-9-fluoro-11-hydroxy-16-methoxyandrosta-1,4-dien-3-one.

11. The 3-ketoandrostene in accordance with claim 2, 17-(ethylthio)-9-fluoro-11β-hydroxy-17-(methylthio)androsta-1,4-dien-3-one.

12. The 3-ketoandrostene in accordance with claim 2, 17-(ethylthio)-9-fluoro-11β-hydroxy-17-(phenylthio)androsta-1,4-dien-3-one.

13. The 3-ketoandrostene in accordance with claim 2, 17-(butylthio)-17-(ethylthio)-9-fluoro-11β-hydroxyandrosta-1,4-dien-3-one.

14. The 3-ketoandrostene in accordance with claim 2, 17α-(ethylthio)-9-fluoro-11β-hydroxy-17-(methylthio)androsta-1,4-dien-3-one.

15. The 3-ketoandrostene in accordance with claim 2, 17β-(ethylthio)-9-fluoro-11β-hydroxy-17-(methylthio)androsta-1,4-dien-3-one.

16. The 3-ketoandrostene in accordance with claim 2, 17α-(ethylthio)-11β-hydroxy-17-(methylthio)-androsta-1,4-dien-3-one.

17. The 3-ketoandrostene in accordance with claim 2, 17β-(ethylthio)-11β-hydroxy-17-(methylthio)-androsta-1,4-dien-3-one.

18. 3-ketoandrostenes as claimed in claim 1 to 17 for the topical use as antiinflammatory agents.

19. 3-ketoandrostenes as claimed in claim 2 and 6 for the topical use as antiinflammatory agents.

20. 3-ketoandrostenes as claimed in claim 3 and 7 for the topical use as antiinflammatory agents.

21. 3-ketoandrostenes as claimed in claim 4 and 8 for the topical use as antiinflammatory agents.

22. 3-ketoandrostenes as claimed in claim 5 and 9 for the topical use as antiinflammatory agents.

23. Pharmaceutical compositions containing a compound according to claim 1 to 17.

**Claims for the Contracting State: AT**

1. A process for preparing 3-ketoandrostene compounds having in the 17-position the substituents $R_1$—S— and $R_2$—S— wherein $R_1$ and $R_2$ are different groups, and each is alkyl, cycloalkyl or aryl which comprises reacting a compound of the formula:

V

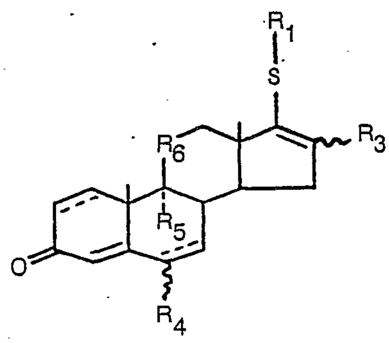

wherein $R_1$ is an alkyl, cycloalkyl or an aryl group;

$R_3$ is hydrogen, hydroxy, alkoxy, aryloxy, alkylthio, arylthio,

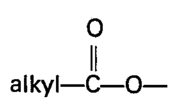

or halogen;

$R_4$ is hydrogen, methyl, hydroxy,

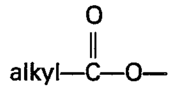

or halogen;

$R_5$ is hydrogen or halogen; and

$R_6$ is carbonyl or β-hydroxymethylene with a compound having the formula

VII $R_2$—SH

in the presence of a Lewis acid such as boron trifluoride etherate wherein $R_2$ is selected from the same groups at $R_1$ but is a different group to yield the compound having the formula:

VIII

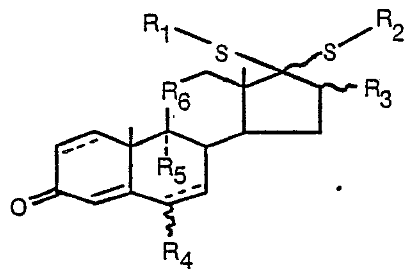

wherein $R_1$, $R_2$, $R_3$, $R_4$, $R_5$ and $R_6$ are as defined above.

2. The process according to claim 1 wherein the reaction is carried out at a temperature of −20°C to −100°C to yield a stereospecific compound having the formula:

IX

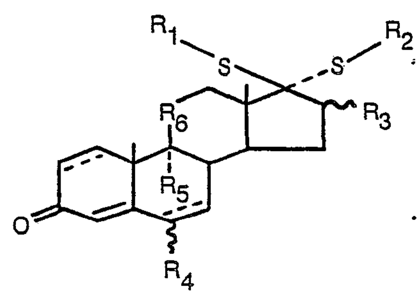

3. A process in accordance with claim 1 in which the compound produced has the formula

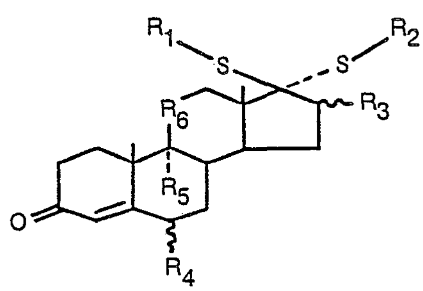

or 1,2-, 6,7- and 15,16-dehydro derivatives thereof, wherein $R_1$ and $R_2$ are different $C_{1-12}$ alkyl, $C_{3-7}$ cycloalkyl, phenyl or phenyl substituted by one or two halogen atoms or one or two $C_{1-12}$ alkyl or alkoxy groups;

$R_3$ is hydrogen, hydroxy, alkoxy, aryloxy, alkylthio, arylthio,

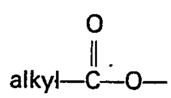

or halogen;

$R_4$ is hydrogen, methyl, hydroxy,

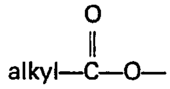

or halogen;

$R_5$ is hydrogen or halogen; and

$R_6$ is carbonyl or β-hydroxymethylene.

4. A process according to claim 3 wherein $R_4$ is hydrogen.

5. A process in accordance with claim 3 wherein $R_5$ is fluorine.

6. A process in accordance with claim 3 wherein $R_6$ is β-hydroxymethylene.

7. The process in accordance with claim 3 wherein the compound produced is 17-(ethylthio)-9-fluoro-11β-hydroxy-17-(methylthio)androsta-1,4-dien-3-one.

8. The process in accordance with claim 3 wherein the compound produced is 17-(ethylthio)-9-fluoro-11β-hydroxy-17-(phenylthio)androsta-1,4-dien-3-one.

9. The process in accordance with claim 3 wherein the compound produced is 17-(butylthio)-17-(ethylthio)-9-fluoro-11β-hydroxyandrosta-1,4-dien-3-one.

10. The process in accordance with claim 3 wherein the compound produced is 17α-(ethylthio)-9-fluoro-11β-hydroxy-17-(methylthio)androsta-1,4-dien-3-one.

11. The process in accordance with claim 3 wherein the compound produced is 17β-(ethylthio)-9-fluoro-11β-hydroxy-17-(methylthio)androsta-1,4-dien-3-one.

12. The process in accordance with claim 3 wherein the compound produced is 17α-(ethylthio)-11β-hydroxy-17-(methylthio)androsta-1,4-dien-3-one.

13. The process in accordance with claim 3 wherein the compound produced is 17β-(ethylthio)-11β-hydroxy-17-(methylthio)androsta-1,4-dien-3-one.

14. A process for preparing compounds having the formula

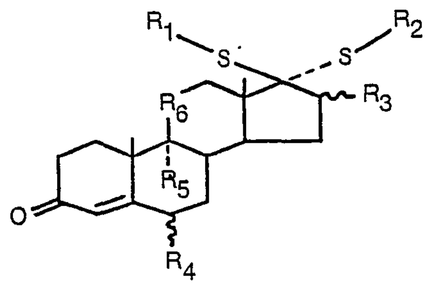

or 1,2-, 6,7- and 15,16-dehydro derivatives thereof, wherein $R_1$ and $R_2$ are the same $C_{1-12}$ alkyl, $C_{3-7}$ cycloalkyl, phenyl or phenyl substituted by one or two halogen atoms or one or two $C_{1-12}$ alkyl or alkoxy groups;

$R_3$ is hydroxy, alkoxy, aryloxy, alkylthio, arylthio,

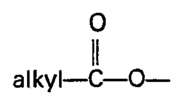

or halogen;

$R_4$ is hydrogen, methyl, hydroxy,

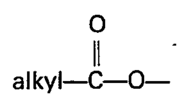

or halogen;

$R_5$ is hydrogen or halogen and

$R_6$ is carbonyl or β-hydroxymethylene which comprises reacting a compound of the formula

II

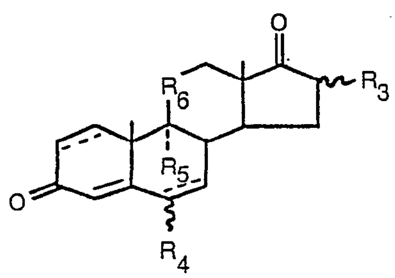

,

with a compound of the formula

III $R_1$—SH

in the presence of a Lewis acid and in the presence of an organic solvent in an inert atmosphere.

15. The process according to claim 14 wherein the reaction is carried out in the additional presence of dimethylformamide dialkyl acetal.

16. The process according to claim 14 wherein the dimethylformamide dialkyl acetal is dimethylformamide dimethyl acetal.

17. A process in accordance with claim 14 wherein $R_4$ is hydrogen.

18. A process in accordance with claim 14 wherein $R_5$ is fluorine.

19. A process in accordance with claim 14 wherein $R_6$ is β-hydroxymethylene.

20. A process in accordance with claim 14 wherein the compound produced is (11β, 16α)-17,17-bis(ethylthio)-9-fluoro-11-hydroxy-16-methoxyandrosta-1,4-dien-3-one.

21. A process according to claim 1 or claim 14 in which $R_6$ is β-hydroxymethylene and the hydroxy group is protected before reaction with a compound of formula III or VII.

22. A process for preparing the compounds of formulae VIII and I having an ethylenic unsaturation in the 15,16 position which comprises refluxing the corresponding 16-haloandrostene in an organic solvent in the presence of 1,5-diazabicyclo (5.4.0) undec-5-ene or dehydrating the corresponding 16-hydroxyandrostene.

**Patentansprüche für die Vertragsstaaten: BE CH DE FR GB IT LI LU NL SE**

1. Ein 3-Ketoandrosten, das in 17-Stellung die Substituenten $R_1$—S— und $R_2$—S— aufweist, in denen $R_1$ und $R_2$ verschiedene Reste sind und jeder einen Alkyl-, Cycloalkyl- oder Arylrest bedeutet.

2. Ein 3-Ketoandrosten nach Anspruch 1 der Formel

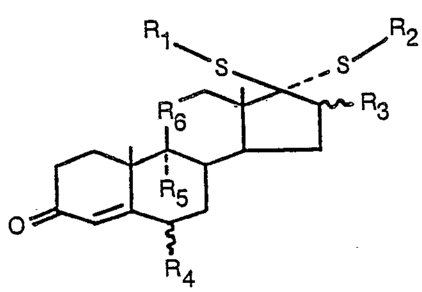

oder 1,2-, 6,7- und 15,16-Dehydro-Derivate davon, in der $R_1$ und $R_2$ verschiedene $C_{1-12}$-Alkyl-, $C_{3-7}$-Cycloalkyl-, Phenyl- oder durch ein oder zwei Halogenatome oder einen oder zwei $C_{1-12}$-Alkyl- oder Alkoxyreste substituierten Phenylrest bedeuten,

$R_3$ ein Wasserstoffatom, einen Hydroxyl-, Alkoxy-, Aryloxy-, Alkythio- oder Arylthiorest, einen Rest

$$\text{Alkyl}—\overset{\overset{\displaystyle O}{\|}}{C}—O—$$

oder ein Halogenatom darstellt;

$R_4$ ein Wasserstoffatom, eine Methyl- oder Hydroxylgruppe, einen Rest

$$\text{Alkyl}—\overset{\overset{\displaystyle O}{\|}}{C}—O—$$

oder ein Halogenatom bedeutet;

$R_5$ ein Wasserstoff- oder Halogenatom darstellt; und

$R_6$ eine Carbonyl- oder β-Hydroxymethylengruppe bedeutet.

3. Ein 3-Ketoandrosten nach Anspruch 2, in dem $R_4$ ein Wasserstoffatom bedeutet.

4. Ein 3-Ketoandrosten nach Anspruch 2, in dem $R_5$ ein Fluoratom bedeutet.

5. Ein 3-Ketoandrosten nach Anspruch 2, in dem $R_6$ eine β-Hydroxymethylengruppe bedeutet.

6. Ein 3-Ketoandrosten der Formel

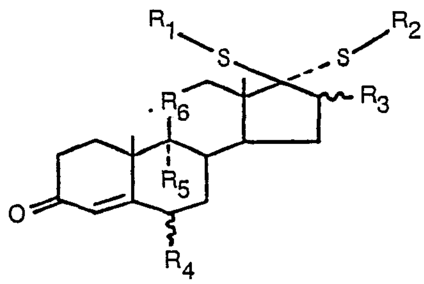

oder 1,2-, 6,7- und 15-16-Dehydro-Derivate davon, in der $R_1$ und $R_2$ die gleichen $C_{1-12}$-Alkyl-, $C_{3-7}$-Cycloalkyl-, Phenyl- oder durch ein oder zwei Halogenatome oder ein oder zwei $C_{1-12}$-Alkyl- oder Alkoxyreste substituierte Phenylgruppe bedeuten;

$R_3$ einen Hydroxyl-, Alkoxy-, Aryloxy-, Alkylthio- oder Arylthiorest, einen Rest

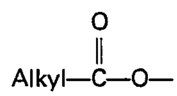

oder ein Halogenatom darstellt;

$R_4$ ein Wasserstoffatom, eine Methyl- oder Hydroxylgruppe, einen Rest

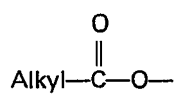

oder ein Halogenatom bedeutet;

$R_5$ ein Wasserstoff- oder Halogenatom darstellt; und

$R_6$ eine Carbonyl- oder β-Hydroxymethylengruppe bedeutet.

7. Ein 3-Ketoandrosten nach Anspruch 6, in dem, $R_4$ ein Wasserstoffatom bedeutet.

8. Ein 3-Ketoandrosten gemäß Anspruch 6, in dem $R_5$ ein Fluoratom bedeutet.

9. Ein 3-Ketoandrosten nach Anspruch 6, in dem $R_6$ eine β-Hydroxymethylengruppe bedeutet.

10. Das 3-Ketoandrosten nach Anspruch 6 (11β,16α)-17,17-Bis-(äthylthio)-9-fluor-11-hydroxy-16-methoxyandrosta-1,4-dien-3-on.

11. Das 3-Ketoandrosten nach Anspruch 2, 17-(Äthylthio)-9-fluor-11β-hydroxy-17-(methylthio)-androsta-1,4-dien-3-on.

12. Das 3-Ketoandrosten nach Anspruch 2, 17-(Äthylthio)-9-fluor-11β-hydroxy-17-(phenylthio)-androsta-1,4-dien-3-on.

13. Das 3-Ketoandrosten nach Anspruch 2, 17-(Butylthio)-17-(äthylthio)-9-fluor-11β-hydroxyandrosta-1,4-dien-3-on.

14. Das 3-Ketoandrosta nach Anspruch 2, 17α-(Äthylthio)-9-fluor-11β-hydroxy-17-(methylthio)-androsta-1,4-dien-3-on.

15. Das 3-Ketoandrosten nach Anspruch 2, 17β-(Äthylthio)-9-fluor-11β-hydroxy-17-(methylthio)-androsta-1,4-dien-3-on.

16. Das 3-Ketoandrosten nach Anspruch 1, 17α-(Äthylthio)-11β-hydroxy-17-(methylthio)-androsta-1,4-dien-3-on.

17. Das 3-Ketoandrosten nach Anspruch 2, 17β-(Äthylthio)-11β-hydroxy-17-(methylthio)-androsta-1,4-dien-3-on.

18. 3-Ketoandrostene nach Anspruch 1 bis 17 zur lokalen Anwendung als entzündungshemmende Wirkstoffe.

19. 3-Ketoandrostene nach Anspruch 2 und 6 zur lokalen Anwendung als entzündungshemmende Wirkstoffe.

20. 3-Ketoandrostene nach Anspruch 3 und 7 zur lokalen Anwendung als entzündungshemmende Wirkstoffe.

21. 3-Ketoandrostene nach Anspruch 4 und 8 zur lokalen Anwendung als entzündungshemmende Wirkstoffe.

22. 3-Ketoandrostene nach Anspruch 5 und 9 zur lokalen Anwendung als entzündungshemmende Wirkstoffe.

23. Arzneimittel, enthaltend eine Verbindung nach Anspruch 1 bis 17.

**Patentansprüche für den Vertragsstaat: AT**

1. Verfahren zur Herstellung von 3-Ketoandrosten-Verbindungen mit den Substituenten $R_1$—S— und $R_2$—S— in der 17-Stellung, wobei $R_1$ und $R_2$ verschiedene Reste sind und jeder einen Alkyl-, Cycloalkyl-oder Arylrest bedeutet, dadurch gekennzeichnet, daß man eine Verbindung der Formel V

V

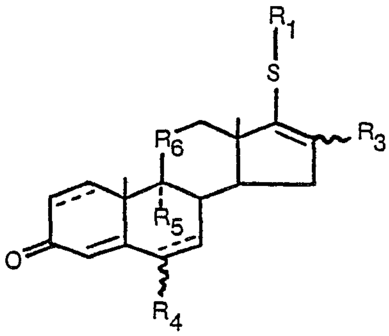

in der $R_1$ einen Alkyl-, Cycloalkyl- oder Arylrest bedeutet, $R_3$ ein Wasserstoffatom, einen Hydroxyl-, Alkoxy-, Aryloxy-, Alkylthio- oder Arylthiorest, einen

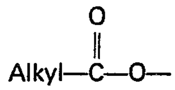

oder ein Halogenatom darstellt;

$R_4$ ein Wasserstoffatom, eine Methyl- oder Hydroxylgruppe, einen Rest

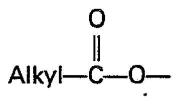

oder ein Halogenatom bedeutet;

$R_5$ ein Wasserstoff- oder Halogenatom darstellt und

$R_6$ eine Carbonyl- oder β-Hydroxymethylengruppe bedeutet,

mit einer Verbindung der Formel VII

$R_2$—SH (VII)

in Gegenwart einer Lewis-Säure, wie Bortrifluorid-ätherat, wobei der Rest $R_2$ aus der gleichen Gruppe gewählt ist wie der rest $R_1$, jedoch ein unterschiedlicher Rest ist, zu einer Verbindung der Formel VIII umsetzt,

VIII

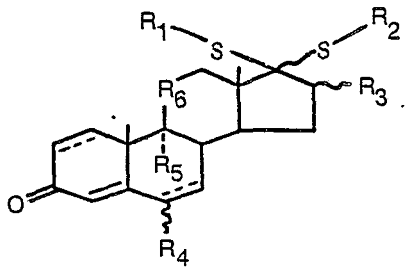

in der $R_1$, $R_2$, $R_3$, $R_4$, $R_5$ und $R_6$ wie vorstehend definiert sind.

2. Verfahren nach Anspruch 1, wobei die Umsetzung bei einer Temperatur von −20 bis −100°C zu einer stereospezifischen Verbindung der Formel IX durchgeführt wird:

IX

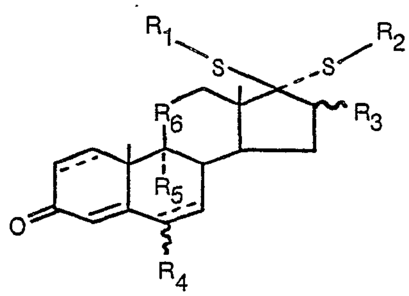

3. Verfahren nach Anspruch 1, wobei die hergestellte Verbindung die Formel

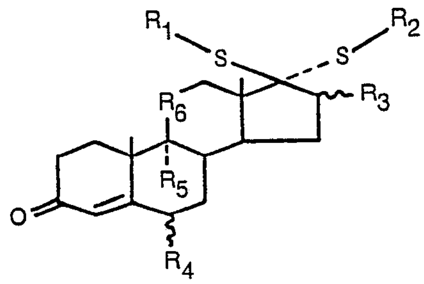

aufweist, oder 1,2-, 7,6- und 15,16-Dehydro-Derivate davon, wobei $R_1$ und $R_2$ verschiedene $C_{1-12}$-Alkyl-, $C_{3-7}$-Cycloalkyl-, Phenyl- oder durch ein oder zwei Halogenatome oder einen oder zwei $C_{1-12}$-Alkyl- oder Alkoxyreste substituierten Phenylrest bedeuten,

$R_3$ ein Wasserstoffatom, einen Hydroxyl-, Alkoxy-, Aryloxy-, Alkylthio- oder Arylthiorest, einen Rest

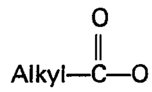

oder ein Halogenatom darstellt;

$R_4$ ein Wasserstoffatom, eine Methyl- oder Hydroxylgruppe, eine Rest

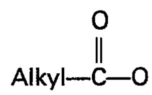

oder ein Halogenatom bedeutet;

$R_5$ ein Wasserstoff- oder Halogenatom darstellt; und

$R_6$ eine Carbonyl- oder β-Hydroxymethylengruppe bedeutet.

4. Verfahren nach Anspruch 3, wobei $R_4$ ein Wasserstoffatom ist.

5. Verfahren nach Anspruch 3, wobei $R_5$ ein Fluoratom ist.

6. Verfahren nach Anspruch 3, wobei $R_6$ eine β-Hydroxymethylengruppe ist.

7. Verfahren nach Anspruch 3, wobei die erzeugte Verbindung 17-(Äthylthio)-9-fluor-11β-hydroxy-17-(methylthio)-androsta-1,4-dien-3-on ist.

8. Verfahren nach Anspruch 3, wobei die erzeugte Verbindung 17-(Äthylthio)-9-fluor-11β-hydroxy-17-(phenylthio)-androsta-1,4-dien-3-on ist.

9. Verfahren nach Anspruch 3, wobei die erzeugte Verbindung 17-(Butylthio)-17-(äthylthio)-9-fluor-11β-hydroxyandrosta-1,4-dien-3-on ist.

10. Verfahren nach Anspruch 3, wobei die erzeugte Verbindung 17α-(äthylthio)-9-fluor-11β-hydroxy-17-(methylthio)-androsta-1,4-dien-3-on ist.

11. Verfahren nach Anspruch 3, wobei die erzeugte Verbindung 17β-(Äthylthio)-9-fluor-11β-hydroxy-17-(methylthio)-androsta-1,4-dien-3-on ist.

12. Verfahren nach Anspruch 3, wobei die erzeugte Verbindung 17α-(Äthylthio)-11β-hydroxy-17-(methylthio)-androsta-1,4-dien-3-on ist.

13. Verfahren nach Anspruch 3, wobei die erzeugte Verbindung 17β-(Äthylthio)-11β-hydroxy-17-(methylthio)-androsta-1,4-dien-3-on ist.

14. Verfahren zur Herstellung von Verbindungen der Formel I

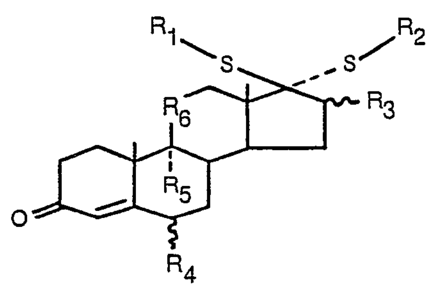

(1)

oder von 1,2-, 6,7- und 15,16-Dehydro-Derivaten davon, wobei $R_1$ und $R_2$ die gleichen $C_{1-12}$-Alkyl-, $C_{3-7}$-Cycloalkyl-, Phenyl- oder durch ein oder zwei Halogenatome oder ein oder zwei $C_{1-12}$-Alkyl- oder Alkoxyreste substituierte Phenylgruppe bedeuten;

$R_3$ einen Hydroxyl-, Alkoxy-, Aryloxy-, Alkylthio- oder Arylthiorest, eine Rest

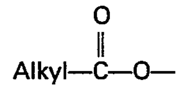

oder ein Halogenatom darstellt;

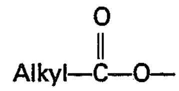

oder ein Halogenatom bedeutet;

$R_5$ ein Wasserstoff- oder Halogenatom darstellt; und

$R_6$ eine Carbonyl- oder β-Hydroxymethylengruppe bedeutet, dadurch gekennzeichnet, daß man eine Verbindung der Formel II

II

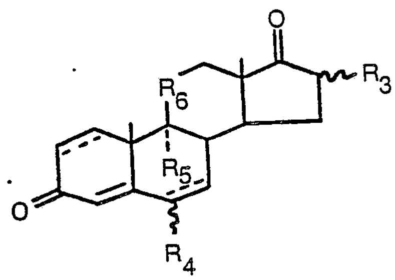

,

mit einer Verbindung der Formel II

$R_1$—SH (III)

in Gegenwart einer Lewis-Säure und in Gegenwart eines organischen Lösungsmittels in einer inerten Atmosphäre umsetzt.

15. Verfahren nach Anspruch 14, wobei die Umsetzung in zusätzlicher Gegenwart von Dimethylformamid-dialkylacetal durchgeführt wird.

16. Verfahren nach Anspruch 14, wobei das Dimethylformamid-dialkylacetal Dimethylformamiddimethylacetal ist.

17. Verfahren nach Anspruch 14, wobei $R_4$ ein Wasserstoffatom bedeutet.

18. Verfahren nach Anspruch 14, wobei $R_5$ ein Fluoratom bedeutet.

19. Verfahren nach Anspruch 14, wobei $R_6$ eine β-Hydroxymethylengruppe bedeutet.

20. Verfahren nach Anspruch 14, wobei die hergestellte Verbindung (11β,16α)-17,17-Bis-(äthylthio)-9-fluor-11-hydroxy-16-methoxyandrosta-1,4-dien-3-on ist.

21. Verfahren nach Anspruch 1 oder Anspruch 14, wobei $R_6$ eine β-Hydroxymethylengruppe ist und die Hydroxylgruppe vor der Umsetzung mit einer Verbindung der Formel III oder VII geschützt wird.

22. Verfahren zur Herstellung der Verbindungen der Formeln VIII und I mit einer äthylenisch ungesättigten Bindung in 15,16-Stellung, dadurch gekennzeichnet, daß man des entsprechende 16-Halogenandrosten in einem organischen Lösungsmittel in Gegenwart von 1,5-Diazabicyclo(5.4.0)undec-5-en unter Rückfluß erhitzt oder das entsprechende 16-Hydroxyandrosten dehydriert.

**Revendications pour les Etats contractants: BE CH DE FR GB IT LI LU NL**

1. 3-cétoandrostène portant en position 17 les substituants $R_1$—S— et $R_2$—S— dans lesquels $R_1$ et $R_2$ sont des groupements différents, et sont chacun un radical alkyle, cycloalkyle ou aryle.

2. 3-cétoandrostène selon la revendication 1, ayant pour formule

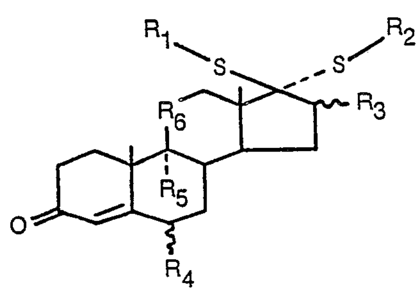

ou dérivés 1,2-, 6,7- et 15,16-déhydro de celui-ci, formule dans laquelle:

$R_1$ et $R_2$ sont différents et sont chacun un radical alkyle en $C_1$—$C_{12}$, cycloalkyle en $C_3$—$C_7$, phényle, ou phényle substitué par un ou deux atomes d'halogène ou par un ou deux radicaux alkyle ou alcoxy en $C_1$—$C_{12}$;

$R_3$ est un atome d'hydrogène, un groupement hydroxy, un radical alcoxy, aryloxy, alkylthio, arylthio ou

$$\text{alkyl}-\overset{\overset{\displaystyle O}{\|}}{C}-O-,$$

ou un atome d'halogène;

$R_4$ est un atome d'hydrogène, un radical méthyle, hydroxy ou

$$\text{alkyl}-\overset{\overset{\displaystyle O}{\|}}{C}-O-,$$

ou un atome d'halogène;

$R_5$ est un atome d'hydrogène ou d'halogène; et

$R_6$ est un groupement carbonyle ou β-hydroxyméthylène.

3. 3-cétoandrostène selon la revendication 2, dans lequel $R_4$ est un atome d'hydrogène.

4. 3-cétoandrostène selon la revendication 2, dans lequel $R_5$ est un atome de fluor.

5. 3-cétoandrostène selon la revendication 2, dans lequel $R_6$ est un groupement β-hydroxyméthylène.

6. 3-cétoandrostène ayant pour formule

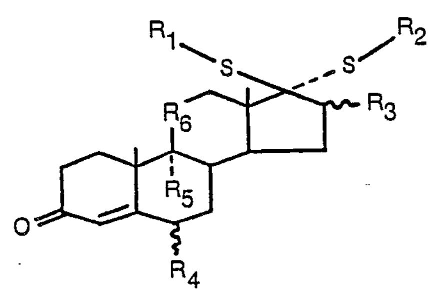

ou dérivés 1,2-, 6,7- et 15,16-déhydro de celui-ci, formule dans laquelle:

$R_1$ et $R_2$ sont identiques et sont chacun un radical alkyle en $C_1$—$C_{12}$, cycloalkyle en $C_3$—$C_7$, phényle, ou phényle substitué par un ou deux atomes d'halogène ou par un ou deux radicaux alkyle ou alcoxy en $C_1$—$C_{12}$;

$R_3$ est un groupement hydroxy, un radical alcoxy, aryloxy, alkylthio, arylthio ou

$$\text{alkyl}-\overset{\overset{\displaystyle O}{\|}}{C}-O-,$$

ou un atome d'halogène;

$R_4$ est un atome d'hydrogène, un radical méthyle, un groupement hydroxy, un groupement

$$\text{alkyl}-\overset{\overset{\displaystyle O}{\|}}{C}-O-$$

ou un atome d'halogène;

$R_5$ est un atome d'hydrogène ou d'halogène; et

$R_6$ est un groupement carbonyle ou β-hydroxyméthylène.

7. 3-cétoandrostène selon la revendication 6, dans lequel $R_4$ est un atome d'hydrogène.

8. 3-cétoandrostène selon la revendication 6, dans lequel $R_5$ est un atome de fluor.

9. 3-cétoandrostène selon la revendication 6, dans lequel $R_6$ est un groupement β-hydroxyméthylène.

10. 3-cétoandrostène selon la revendication 6, qui est la (11β,16α)-17,17-bis(éthylthio)-9-fluro-11-hydroxy-16-méthoxyandrosta-1,4-dién-3-one.

11. 3-cétoandrostène selon la revendication 2, qui est la 17-(éthylthio)-9-fluoro-11β-hydroxy-17-(méthylthio)androsta-1,4-dién-3-one.

12. 3-cétoandrostène selon la revendication 2, qui est la 17-(éthylthio)-9-fluoro-11β-hydroxy-17-(phénylthio)androsta-1,4-dién-3-one.

13. 3-cétoandrostène selon la revendication 2, qui est la 17-(butylthio)-17-(éthylthio)-9-fluoro-11β-hydroxyandrosta-1,4-dién-3-one.

14. 3-cétoandrostène selon la revendication 2, qui est la 17α-(éthylthio)-9-fluoro-11β-hydroxy-17-(méthylthio)androsta-1,4-dién-3-one.

15. 3-cétoandrostène selon la revendication 2, qui est la 17β-(éthylthio)-9-fluoro-11β-hydroxy-17-(méthylthio)androsta-1,4-dién-3-one.

16. 3-cétoandrostène selon la revendication 2, qui est la 17α-(éthylthio)-11β-hydroxy-17-(méthylthio)-androsta-1,4-dién-3-one.

17. 3-cétoandrostène selon la revendication 2, qui est la 17β-(éthylthio)-11β-hydroxy-17-(méthylthio)-androsta-1,4-dién-3-one.

18. 3-cétoandrostènes tels qu'ils sont revendiqués dans les revendications 1 à 17, pour utilisation topique comme agents anti-inflammatoires.

19. 3-cétoandrostènes tels qu'ils sont revendiqués dans les revedications 2 et 6, pour utilisation topique comme agents anti-inflammatoires.

20. 3-cétoandrostènes tels qu'ils sont revendiqués dans les revendications 3 et 7, pour utilisation topique comme agents anti-inflammatoires.

21. 3-cétoandrostènes tels qu'ils sont revendiqués dans les revendications 4 et 8, pour utilisation topique comme agents anti-inflammatoires.

22. 3-cétoandrostènes tels qu'ils sont revendiqués dans les revendications 5 et 9, pour utilisation topique comme agents anti-inflammatoires.

23. Compositions pharmaceutiques contenant un composé selon les revendications 1 à 17.

**Revendications pour l'Etat contractant: AT**

1. Procédé de préparation de 3-cétoandrostènes portant en position 17 les substituants $R_1$—S— et $R_2$—S— dans lesquels $R_1$ et $R_2$ sont des groupements différents et sont chacun un radical alkyle, cycloalkyle ou aryle, qui consiste à faire réagir un composé de formule

V

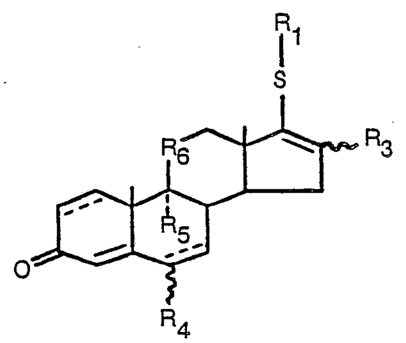

dans laquelle $R_1$ est un radical alkyle, cycloalkyle ou aryle;

$R_3$ est un atome d'hydrogène, un groupement hydroxy, un radical alcoxy, aryloxy, alkylthio, arylthio ou

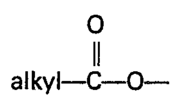

ou un atome d'halogène;

$R_4$ est un atome d'hydrogène, un radical méthyle, un groupement hydroxy, un radical

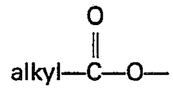

ou un atome d'halogène;

$R_5$ est un atome d'hydrogène ou d'halogène; et

$R_6$ est un groupement carbonyle ou β-hydroxyméthylène, avec un composé de formule

VII $R_2$—SH

en présence d'un acide de Lewis tel que l'éthérat de trifluorure de bore, formule dans laquelle $R_2$ est choisi parmi les mêmes groupements que $R_1$ mais est un groupement différent, pour obtenir le composé de formule

VIII

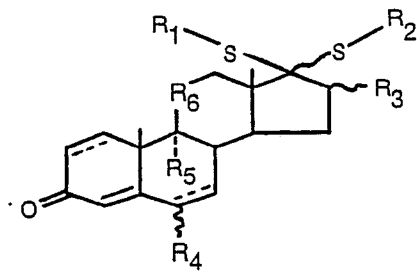

dans laquelle $R_1$, $R_2$, $R_3$, $R_4$, $R_5$ et $R_6$ sont tels que définis ci-dessus.

2. Procédé selon la revendication 1, dans lequel on mène la réaction à une température de −20°C à −100°C pour obtenir un composé stéréo-spécifique ayant pour formule:

IX

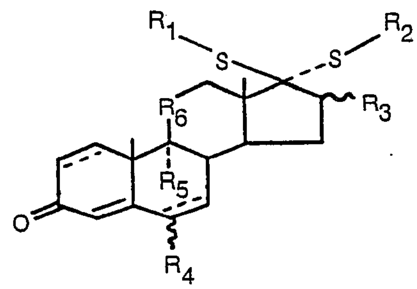

3. Procédé selon la revendication 1, dans lequel le composé obtenu a pour formule

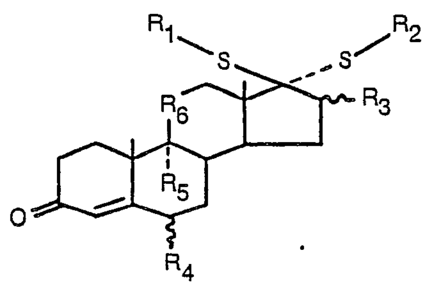

ou est un dérivé 1,2-, 6,7-ou 15,16-déhydro du même composé, formule dans laquelle $R_1$ et $R_2$ sont différents et sont chacun un radical alkyle en $C_1$—$C_{12}$, cycloalkyle en $C_3$—$C_7$, phényle, ou phényle substitué par un ou deux atomes d'halogène ou par un ou deux radicaux alkyle ou alcoxy en $C_1$—$C_{12}$;

$R_3$ est un atome d'hydrogène, un groupement hydroxy, un radical alcoxy, aryloxy, alkylthio, arylthio ou

$$\text{alkyl}—\overset{\overset{\displaystyle O}{\|}}{C}—O—,$$

ou un atome d'halogène;

$R_4$ est un atome d'hydrogène, un radical méthyle, un groupement hydroxy, un radical

$$\text{alkyl}—\overset{\overset{\displaystyle O}{\|}}{C}—O—$$

ou un atome d'halogène;

$R_5$ est un atome d'hydrogène ou d'halogène; et

$R_6$ est un groupement carbonyle ou β-hydroxyméthylène.

4. Procédé selon la revendication 3, dans lequel $R_4$ est un atome d'hydrogène.

5. Procédé selon la revendication 3, dans lequel $R_5$ est un atome de fluor.

6. Procédé selon la revendication 3, dans lequel $R_6$ est un groupement β-hydroxyméthetylène.

7. Procédé selon la revendication 3, dans lequel le composé obtenu est la 17-(éthylthio)-9-fluoro-11β-hydroxy-17-(méthylthio)androsta-1,4-dién-3-one.

8. Procédé selon la revendication 3, dans lequel le composé obtenu est la 17-(éthylthio)-9-fluoro-11β-hydroxy-17-(phénylthio)androsta-1,4-dién-3-one.

9. Procédé selon la revendication 3, dans lequel le composé obtenu est la 17-(butylthio)-17-(éthylthio)-9-fluoro-11β-hydroxyandrosta-1,4-dién-3-one.

10. Procédé selon la revendication 3, dans lequel le composé obtenu est la 17α-(éthylthio)-9-fluoro-11β-hydroxy-17-(méthylthio)androsta-1,4-dién-3-one.

11. Procédé selon la revendication 3, dans lequel le composé obtenu est la 17β-(éthylthio)-9-fluoro-11β-hydroxy-17-(méthylthio)androsta-1,4-dién-3-one.

12. Procédé selon la revendication 3, dans lequel le composé obtenu est la 17α-(éthylthio)-11α-hydroxy-17-(méthylthio)androsta-1,4-dién-3-one.

13. Procédé selon la revendication 3, dans lequel le composé obtenu est la 17β-(éthylthio)-11β-hydroxy-17-(méthylthio)androsta-1,4-dién-3-one.

14. Procédé de préparation de composés ayant pour formule

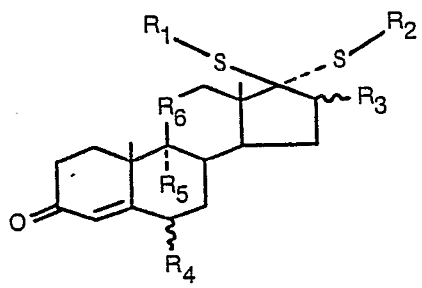

ou de leurs dérivés 1,2-, 6,7-et 15,16-déhydro, formule dans laquelle $R_1$ et $R_2$ sont identiques et sont chacun un radical alkyle en $C_1—C_{12}$, cycloalkyle en $C_3—C_7$, phényle, ou phényle substitué par un ou deux atomes d'halogène ou par un ou deux radicaux alkyle ou alcoxy en $C_1—C_{12}$;

$R_3$ est un groupement hydroxy, un radical alcoxy, aryloxy, alkylthio, arylthio ou

$$\text{alkyl}—\overset{\overset{\displaystyle O}{\|}}{C}—O—$$

ou un atome d'halogène;

$R_4$ est un atome d'hydrogène, un radical méthyle, un groupement hydroxy, un radical

$$\text{alkyl}—\overset{\overset{\displaystyle O}{\|}}{C}—O—$$

ou un atome d'halogène;

$R_5$ est un atome d'hydrogène ou d'halogène et

$R_6$ est un groupement carbonyle ou β-hydroxyméthylène,

qui consiste à faire réagir un composé de formule

II

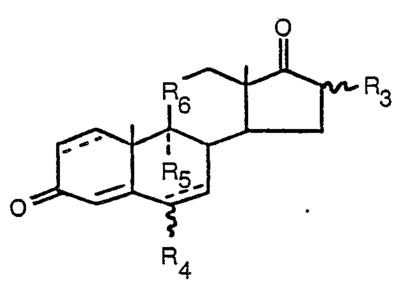

avec un composé de formule

III $R_1—SH$

en présence d'un acide de Lewis et en présence d'un solvant organique dans une atmosphère inerte.

15. Procédé selon la revendication 14, dans lequel on mène la réaction en la présence supplémentaire de dialkylacétal de diméthylformamide.

16. Procédé selon la revendication 14, dans lequel le dialkylacétal de diméthylformamide est le diméthylacétal de diméthylformamide.

17. Procédé selon la revendication 14, dans lequel $R_4$ est un atome d'hydrogène.

18. Procédé selon la revendication 14, dans lequel $R_5$ est un atome de fluor.

19. Procédé selon la revendication 14, dans lequel $R_6$ est un groupement β-hydroxyméthylène.

20. Procédé selon la revendication 14, dans lequel le composé obtenu est la (11β,16α)-17,17-bis(éthylthio)-9-fluoro-11-hydroxy-16-méthoxyandrosta-1,4-dién-3-one.

21. Procédé selon la revendication 1 ou la revendication 14, dans lequel $R_6$ est un groupement β-hydroxyméthylène et on protège le groupement hydroxy avant la réaction avec un composé de formule III ou VII.

22. Procédé de préparation des composés de formules VIII et I ayant une insaturation éthylénique en position 15,16, qui consiste à mettre au reflux le 16-haloandrostène correspondant dans un solvant organique en présence de 1,5-diazabicyclo (5.4.0) undéc-5-ène, ou à déshydrater le 16-hydroxyandrostène correspondant.